# EUROPÄISCHE PATENTSCHRIFT

(11) **EP 0 637 964 B1**
(45) Veröffentlichungstag und Bekanntmachung des Hinweises auf die Patenterteilung: **18.09.1996**
(21) Anmeldenummer: 90908197.8
(22) Anmeldetag: 31.05.1990
(51) Int. Cl.: A61K 45/05, A61K 38/36, A61K 35/78, A61K 38/00, G01N 33/53

(54) **MEDIKAMENTE ZUR TUMORTHERAPIE MIT KONTROLLIERTEM UND REGULIERTEM IMMUNSYSTEM SOWIE VERWENDUNG DER EINZELSUBSTANZEN ZUR KOMBINIERTEN THERAPIE**
DRUGS FOR TUMOUR THERAPY IN CONJUNCTION CONTROL WITH AND REGULATION OF THE IMMUNE SYSTEM AND USE OF THE INDIVIDUAL SUBSTANCES FOR COMBINED THERAPY
MEDICAMENT POUR LE TRAITEMENT DES TUMEURS AVEC CONTROLE ET REGULATION DU SYSTEME IMMUNITAIRE; UTILISATION DES DIFFERENTES SUBSTANCES CONCERNEES DANS LE CADRE D'UNE THERAPIE COMBINEE

(30) Priorität: 01.06.1989 DE 3917852
(43) Veröffentlichungstag der Anmeldung: 15.02.1995
(73) Patentinhaber: Bartos Patent Development and Holding Company Limited, Dublin 2 (IE); BARTOS, Stefan, Dr., D-42697 Solingen (DE)
(72) Erfinder: BARTOS, Stefan, D-5650 Solingen 1 (DE)
(74) Vertreter: Werner, Hans-Karsten, Dr.Dipl.-Chem.
(86) Internationale Anmeldenummer: EP9000868
(87) Internationale Veröffentlichungsnummer: WO9014843

(56) Entgegenhaltungen:
- DE-A- 3 825 186
- Experientia, Bd 26, Nr. 5, 15.05.70, J. Nienhaus et al. S. 523-525.

## Beschreibung

Gegenstand der vorliegenden Erfindung sind Kombinationspräparate zur Tumortherapie mit kontrolliertem und reguliertem Immunsystem sowie die Verwendung entsprechender Einzelsubstanzen zur Herstellung von Medikamenten zur kombinierten Anwendung bei der Tumortherapie mit kontrolliertem und reguliertem Immunsystem. Weiterhin betrifft die Erfindung die Methode zur Tumortherapie mit kontrolliertem und reguliertem Immunsystem durch kombinierte Applikation von wirksamen Substanzen und ein Verfahren zur verbesserten Kontrolle des Immunsystems vor und während der Tumortherapie, insbesondere mit Hilfe der erfindungsgemäß verwendeten Wirkstoffe.

In den letzten Jahren ist das Wissen um das Vorhandensein und die Wirkungsweise des Immunsystems ständig gewachsen. Dabei hat sich die Erkenntnis durchgesetzt, daß sehr viele Krankheiten immunologisch bedingt sind und sich sehr viele Krankheiten mit immunologischen Methoden analysieren und therapeutisch günstig beeinflussen oder sogar heilen lassen.

Die Immunologie ist eine relativ junge Wissenschaft, die sich in einer sehr raschen Entwicklung befindet. Ein guter Überblick über den derzeitigen Stand der Technik der Immunologie gibt "Das Kurze Lehrbuch der Immunologie" von Ivan Roitt, Jonathan Brostoff, David K. Male, sowie die deutsche Übersetzung von Ihor Harabacz, Georg Thieme Verlag 1987, Stuttgart/New York, ISBN 3-13-702101-4.

Auch das Gebiet der Tumorimmunologie ist in den letzten Jahren intensiv bearbeitet worden, wobei jedoch therapeutisch erst geringe Erfolge zu verzeichnen sind. Die Problematik und Komplexität des Sachverhalts geht unter anderem hervor aus dem Buch "Immunologie und Tumormarker beim Mammakarzinom", Herausgeber U.D. Koenig, Ferdinand Enke-Verlag - Stuttgart - 1933, ISBN 3-432-93471-8. Brauchbare und vereinfachte Wege der vorbereitenden und begleitenden immunologischen Untersuchung befinden sich in den Patentanmeldungen des gleichen Anmelders für die "Verfahren zur integrierten onkologischen Eingangsuntersuchung und Krebstherapie", DE-OS 37 19 698 und WO 88/09935 (PCT/EP88/00509).

Hierin ist unter anderem festgestellt, daß die tumorassoziierte Immunreaktion kein Privileg hat. Vereinfacht kann gesagt werden, daß der erste Schritt bei der Auseinandersetzung des Tumorträgers mit den Krebszellen ebenfalls auf der Ebene der Makrophagen und der Natural-Killer-Zellen und dann im Zusammenwirken mit den funktionell verschiedenen Subpopulationen der T-Zellen stattfindet. Erst danach tritt eine B-Zell-Immunantwort auf, mit dem Erscheinen und der Nachweisbarkeit von tumorassoziierten Antikörpern, in der Regel mit den Immunglobulin-Typen Ig-M, Ig-A und Ig-G.

Die tumorassoziierten Antikörper können wiederum die zelluläre Zytotoxizität gegenüber den Tumorzellen verstärken. Jedem Experten ist es bekannt, daß die höchste Effizienz einer antikörpergesteuerten, komplementabhängigen, zellulären Zytotoxizität zuzuordnen ist, wo Antikörper des Typs Ig-M und/oder Ig-G nachweisbar sind.

Erst die Klärung der Qualität und der Stärke einer tumorassoziierten zellulären und humoralen Immunität gibt endgültig darüber Aufschluß, welches zusätzliche prognostische Kriterium die tumorassoziierte Immunreaktion darstellt und welches zusätzliche therapeutische Potential diese beinhaltet.

So wird in der Krebstherapie unverweigerlich die Forderung nach routinemäßig durchführbaren immunbiologischen Eingang- und Kontrolluntersuchungen gestellt. Eine blinde, unkontrollierte spezifische Immuntherapie oder eine einfache unspezifische Reiztherapie mit immunmodulierenden Substanzen ist strikt abzulehnen und wird aus mehreren Gründen als gefährlich bezeichnet. Jede biologische Therapie ist eine zweischneidige Waffe, die sowohl gewünschte als auch ungewünschte Effekte bei dem gleichen Patienten bewirken kann.

So kann man heute mit verschiedenen Untersuchungstechniken sowohl eine reine zelluläre, wie eine reine humorale Immunantwort, oder eine antikörpergesteuerte zelluläre Zytotoxizität messen, und zwar als solche, wie auch gegen die Krebszellen gerichtete tumorassoziierte Immunreaktion, beispielsweise nach der oben genannten Methode gemäß WO 88/09935 ex-vivo-in-vitro. Diesen Untersuchungsergebnissen können sogar heute schon hochsignifikante prognostische Aussagen zugeordnet werden; vergl. U.D. Koenig im Band 12 der Klinik der Frauenheilkunde, Spezielle Onkologie II, Seite 281 bis 326, ISBN 3-541-1520-X.

Es ist das Verdienst von U.D. Koenig und seiner Arbeitsgruppe erkannt zu haben, daß der Leukozytenmigrationsinhibitionstest (LMI) nicht nur ein lehrbuchmäßig anerkanntes Untersuchungsverfahren zum Nachweis einer T-zellabhängigen, adoptiven, zellulären Immunität darstellt, sondern daß beide Reaktionsformen des LMI-Tests, nämlich eine signifikante Migrationshemmung (Inhibition) und eine signifikante Migrationsbeschleunigung (Enhancement) als positive Reaktionsformen - neben der negativen Reaktion - betrachtet werden müssen, und daß diese dann bei dem gleichen Patienten in Abhängigkeit des Krankheitszustandes, der allgemeinen Immunabwehr und der therapeutischen Maßnahmen, wie Chemotherapie und/oder Bestrahlung, ineinander übergehen können.

Die Migrationsbeschleunigung (Enhancement) ist mit Immuntoleranz gleichzusetzen und bedeutet soviel, daß die Immunabwehr die Kontrolle über das Krebswachstum verloren hat. Dieser Zustand ist mit einer sehr schlechten Prognose verbunden und stellt beim Mammakarzinom de facto ein Todesurteil für die Patentien dar. Angelsächsische Autoren, wie Zachrau et al. haben diese Tatsache bisher übersehen, indem sie die LMI-Reaktionsform Enhancement nicht als positive Reaktion erkannt haben, sondern von der Annahme ausgingen, daß beim metastasierenden Krebs die Tumorzellen ihre Antigenität verloren hätten und aus diesem Grunde die Tochtergeschwulste entstehen könnten, da ja die positive Reaktion (Inhibition) im LMI-Test nicht mehr feststellbar ist. Es ist hingegen zu vermerken, daß man beim metastasierenden Krebs und auch beim Mammakarzinom ausschließlich die Befundkonstellation Enhancement im LMI-Test vorfindet. Wie wichtig diese Erkenntnis für die Wertung von Inhibition und Enhancement als positive Reaktionsformen des LMI-Testes mit unterschiedlicher prognostischer, immunologischer Aussage ist, kann anhand einer kürzlichen wissenschaftlichen Publikation belegt werden: D. Jenkins, H.O. Douglass, Jr., M. H. Goldrosen: "Role of T-Cells in the Human Leukocyte Adherence Inhibition Assay", Tumor Diagnostik & Therapie 8 (1987), Seiten 204 bis 209.

Der Leukozyten-Adhärenz-Inhibitionstest (LAI) stellt ebenfalls eine anerkannte Methode zum Nachweis einer Immunantwort dar. Zwar beschreiben die Autoren eine komplizierte in-vitro-Versuchsanordnung, die erkennen läßt, welche Bedeutung die Supressor-T-Zellen im Reaktionsausfall des LAI-Testes spielen. Die Autoren zeigen sogar tabellarisch gehemmte (Inhibition) und vermehrte (Enhancement) Adhärenz der Leukozyten, erkennen aber nicht, daß auch die vermehrte Adhärenz, wie die beschleunigte Reaktion im LMI-Test, als positive Reaktion gewertet werden muß. Sobald die Autoren die Supressor-T-Zellen aus der Population der isolierten Abwehrzellen weglysierten, stellte sich auch an negativen (Enhancement)-Proben eine Inhibition der Adhärenz und damit eine positive LAI-Reaktion ein.

Dies sei ein Hinweis darauf, daß die zwei positiven Reaktionsformen der LMI-Testes im Sinne der Inhibition und Enhancement kein Privileg des Leukozytenmigrationsinhibitionstestes sind, sondern daß hier eine allgemein gültige Erkenntnis vorliegt. So könnte man zum Monitoring der tumorassoziierten immunologischen Reaktion im Rahmen der Krebserkrankung als qualitativen und quantitativen ex-vivo-in-vitro Test nicht nur den LMI-Test, sondern auch den LAI-Test heranziehen. Aus praktisch-pragmatischen Gründen wird jedoch der LMI-Test angewendet, da mit diesem Testsystem seit 20 Jahren mehrere unabhängige Arbeitsgruppen die gleichen Resultate und Aussagen erzielt haben und daher als international wissenschaftlichschulmedizinisch abgesichert gelten.

Aus der oben erwähnten WO 88/09935 geht hervor, welche Bedeutung die Etablierung eines autologen Kontroll- und Testsystems für die Tumorimmunologie hat. Gleichzeitig wird aufgezeigt, wie man rationell aus patienteneigenem Tumorgewebe/-material autologes Antigen/Vakzine für tumor-immunbiologische ex-vivo-in-vitro Eingangs- und Kontrolluntersuchungen in biologisch nativer Form durchführen kann, und zwar sogar in Hauttest- und Vakzinen-Qualität präpariert.

Wesentlich dabei ist, daß durch den parallelen Einsatz der lösbaren Antigenfraktion (Cytosol) und der nicht lösbaren Antigenfraktion (Tumorsediment/Zellmembran) das volle Antigenspektrum einer Tumorzelle erfaßt und abgedeckt wird, und zwar in der Weise, daß dann sogar in Abhängigkeit der Reaktionsausfälle differenziert werden kann, wo die Hauptmenge der Antigenität sitzt, nämlich im Zellinneren oder in der Zellmembran. Diese Aussagen werden bestätigt durch erste Ergebnisse am Beispiel des Mamma- und Ovarialkarzinoms (Stefan Janke und Mitarbeiter: Vortrag am 6. Arbeitsgespräch zur Tumorimmunologie in der Gynäkologischen Onkologie am 12. Juni 1988 in Homburg an der Saar und J. Reinsberg und Mitarbeiter: Carinoembrionic antigen (CEA), CA-15-3 und TPA in the cytosol of breast cancer patients, Vortrag am 7. Arbeitsgespräch zur Tumorimmunologie in der Gynäkologischen Onkologie am 9. und 10. Dezember 1988 in Heidelberg, Publikationen in Vorbereitung bzw. im Druck).

Über die Cytosol-CEA-Bestimmung lassen sich drei Grundtypen des Mammakarzinoms differenzieren: Gruppe 1 (Typ-O), Gruppe 2 (Typ-A) und Gruppe 3 (Typ-B). Die Cytosol-CEA-Konzentration per mg korrigiertem Tumorprotein stellt zwischen 0,0 bis 0,25 und zwischen 0,25 bis 4,0 sowie über 4,0 ng CEA/mg korrigiertem Tumorprotein drei Verteilungskurven dar, die jeweils einen Biotyp des Mammakarzinoms repräsentieren. Gruppe 2 (Biotyp-A) Mammakarzinompatientinnen zeigen hochsignifikant gehäuft, bis zu 49 %, eine immunologische Kreuzreaktion im LMI-Test gegen das Hüllprotein gp 55 des Maus-Mammatumor-Virus (MMTV) Typ Paris III. Demgegenüber reagieren gegen gp 55 Mammakarzinompatientinnen aus der Gruppe 1 und 3 nur bis 11 % bis 19 %. Aus diesem Grunde und anhand anderer immunologischer-virologischer Daten wird der Biotyp-A des Mammakarzinoms auch als retrovirusassoziiertes Mammakarzinom bezeichnet.

Es ist eine interessante Erkenntnis, daß die Hauptmenge der Antigenität beim Biotyp A des Mammakarzinoms aus dem Zellinneren stammt, d.h. aus dem lösbaren Cytosol-Anteil, und es häufig vorkommt, daß die Antigenität gegen die Zellmembranfraktion bei diesem Biotyp bereits verloren gegangen ist und im LMI-Test nicht mehr feststellbar ist, obwohl gegen die Cytosol-Antigenfraktion noch eine starke Reaktion zu registrieren ist. Dieses Phänomen ist sehr wichtig, denn Abwehrzellen können vitale Tumorzellen erst dann als fremd erkennen und angreifen, wenn die Antigenität in der Zellmembran lokalisiert ist. Demgegenüber findet man beim Biotyp-B des Mammakarzinoms und beim Ovarialkarzinom stets eine Antigenität sowohl in der Zellmembran wie in der Cytosolfraktion. Diese differente Reaktion wird damit erklärt, daß beim retrovirusassoziierten Mammakarzinom die Tumorantigenität und die Tumorantigene mit hoher Wahrscheinlichkeit Genprodukte eines Virusgenoms sind bzw. dadurch bedingt sind. Demgegenüber findet man beim Ovarialkarzinom und beim Biotyp-B des Mammakarzinoms vorwiegend Entdifferenzierungsantigene in der Zellmembran.

Durch Antigenpräparation gemäß WO 88/09935 am Beispiel des Mammakarzinoms für den LMI-Test wurde festgestellt, daß es dringend zu empfehlen ist, mit mehreren verschieden großen Antigendosen zu testen, um die T-zellabhängige, adoptive, tumorassoziierte Immunität ex-vivo-in-vitro zu messen. Verwendet wurden hierzu jeweils drei Antigendosen, und zwar vorzugsweise mit 100, 20 und 4 Mikrogramm korrigiertem Cytosolprotein per ml Medium und 100, 20 und 4 Mikroliter Standard-Tumorsediment-0,2 mü-Filtrat-Präparation per ml LMI-Medium. Hierbei wurde eine erhöhte Positivitätsrate des LMI-Testes bezüglich Inhibition und Enhancement gefunden, und zwar von 39,6 % (21/53) bei einfacher Testung auf 77,3 % (34/44), wenn mindestens zwei Cytosol-Antigendosen und drei Tumorsediment Antigendosen eingesetzt wurden. Es wurden 78,3 % (18/23) gefunden, wenn der Test komplett jeweils mit drei Cytosol-Antigen und drei Tumorsediment Antigendosen durchgeführt werden konnte. Der Unterschied in der Positivitätsrate ist hochsignifikant (p kleiner als 0,001).

Diese Verbesserung der Empfindlichkeit des LMI-Testes durch den Einsatz von mehreren, vorzugsweise von drei geeigneten Antigendosen, ist beim Mammakarzinom äußerst wichtig, da hierdurch bereits zum Zeitpunkt der Primärtherapie durch ein einziges Testverfahren praktisch alle immunologisch aktiven Mammakarzinome erfaßt werden können. Beim klinisch manifest metastasiertem Mammakarzinom kann man mit Kombination von verschiedenen immunologischen Verfahren maximal in 75,3 % der Fälle zirkulierende Immunkomplexe nachweisen, d.h. eine immunologische Auseinandersetzung des Körpers mit der Krebskrankheit (Krapf, F.D. und Mitarbeiter: "Nachweis von zirkulierenden Immunkomplexen bei Mammakarzinom und malignen Melanom mit drei verschiedenen Methoden", Tumordiagnostik & Therapie 3 (1982), Seite 219.

In einer früheren Arbeit hat der Erfinder zeigen können, daß man mit Hilfe der indirekten Immunfluoreszenz bereits zum Zeitpunkt der Primärtherapie an Mammakarzinomzellen in 75 % der Fälle (21/28) freie, tumorassoziierte Antikörper des Types IgG, IgM und IgA nachgewiesen werden können; vergl. D.S. Bartos: "Tumorassoziierte IgG-, IgM- und Ig-A-Antikörper an Mammakarzinomzellen und ihre mögliche prognostische Bedeutung", Immunologie und Tumormarker beim Mammakarzinom, herausgegeben von U.D. Koenig, Ferdinand-Elke-Verlag, 1983/Stuttgart, ISBN 3-432-93471-8), Seiten 37 bis 43. Danach findet man Ig-A-Antikörper in 61 % (17/28) IgM-Antikörper in 35 % (7/20) und IG-G-Antikörper in 22 % (4/18) aller Fälle. Damit steht beispielsweise fest, daß in der überwiegenden Mehrzahl der Brustkrebsfälle bereits zum Zeitpunkt der Diagnose und Primärtherapie eine antikörpergesteuerte zelluläre Immunantwort/Zytotoxizität vorliegt und nicht nur eine rein zelluläre, tumorassoziierte Reaktion. Weiterhin ergibt sich hieraus, daß im LMI-Test sowohl eine zelluläre T-zellabhängige Immunreaktion erfaßt wird als auch eine antikörpergesteuerte zelluläre Immunantwort.

Lebende Tumorzellen, insbesondere beim Mammkarzinom, routinemäßig zu isolieren und weiteren Untersuchungen zuzuführen, ist technisch sehr schwierig und kostenaufwendig. Andererseits ist es von immenser prognostischer und therapeutischer Bedeutung, mindestens qualitativ zu wissen, welche Art und Stärke einer tumorassoziierten Immunreaktion vorliegt. Aus diesem Grunde wurde der Versuch am Beispiel des Mammakarzinoms unternommen, die tumorassoziierten Antikörper an Mammakarzinomzellen einfach immunhistochemisch an Paraffin-Schnitten nachzuweisen, da diese routinemäßig in der histopathologischen Diagnostik angefertigt werden. Diese Ergebnisse wurden ebenfalls am 7. Arbeitsgespräch zur Tumorimmunologie in der Gynäkologischen Onkologie am 9. und 10. Dezember 1988 in Heidelberg vorgetragen (S. Janke und Mitarbeiter: "Immunhistochemical detection of immunglobulin classes IgG, IgM,IgA and T-cell-dependent tumorassociated immunoreaction in breast cancer patients". Die Ergebnisse waren bei dieser Technik weitgehend identisch: In 68,4 % der Fälle (13/19) fand man Mammakarzinomzellen tumorassoziierte Antikörper, in 57,8 % der Fälle (11/19) IgA und in 42,1 % der Fälle (8/19) IgM und in 31,6 % der Fälle (6/19) IgG.

Es gehört zur Probedeutik der Immunologie, daß IgA-Antikörper immunbiologisch nicht so aktiv und wirksam sind wie IgM- und/oder IgG-Antikörper. So ist die effektivste antikörpergesteuerte zelluläre Zytotoxizität allgemein und tumorassoziiert einer Immunreaktion zuzuordnen, die durch komplementabhängige IgG-Antikörper bewirkt und kontrolliert wird.

Die tumorassoziierte Immunabwehr läßt sich medikamentös mit therapeutischer Absicht in mannigfacher Art und Weise beeinflussen und verstärken. Ein zufriedenstellender therapeutischer Erfolg wird sich jedoch immer erst dann einstellen, wenn die obigen Ausführungen und Kriterien mitberücksichtigt werden.

Liegt eine antikörpergesteuerte zelluläre Zytotoxizität vor, die sogar durch komplementabhängige, tumorassoziierte Antikörper des Typs Ig-G bewirkt wird, welche eine der effektivsten Immunabwehrreaktionen darstellt, dann wird man zur Therapie und Verstärkung der tumorassoziierten Immunreaktion und zum Durchbrechen einer Immuntoleranz im Falle einer Tumorprogression/Rückfall nicht eine immunstimulierende Substanz oder Stoffkombination einsetzen, wie z.B. Xyloglucan und Arabinogalaktan, die aus den oberirdischen Teilen der Heilpflanze Echinacea isoliert werden. Stattdessen setzt man eine immuntherapeutische Maßnahme ein, die die antikörpergesteuerte zelluläre Zytotoxizität so verstärkt, daß möglichst sogar die Immuntoleranz durchbrochen wird und im LMI/LAI-Test statt Enhancement erneut eine Migrationshemmung/Inhibition feststellbar wird. Erst in diesem Fall kann klinisch ein therapeutischer Effekt erwartet werden. Die Wirkstoffe der Echinacea-Pflanze können diese Aufgabe nicht erfüllen, wie auch klinisch belegt werden konnte, da die beiden Polysaccharide Xyloglucan und Arabinogalaktan ausschließlich auf der untersten Ebene eine Immunantwort der Abwehrzellen stimulieren: Die Makrophagen und die Granulozyten. Xyloglucan stimuliert speziell die weißen Blutkörperchen (Granulozyten) und die Makrophagen (große Freßzellen). Arabinogalaktan zeigt die gleiche Wirkung, allerdings im wesentlich geringeren Maße. Dieser Wirkstoff stimuliert jedoch zusätzlich die Makrophagen zur Freisetzung des Tumornekrosefaktors (TNF). Zur Zeit werden von der Pharmaindustrie große Anstrengungen gemacht, gentechnologisch hergestellte natürlich Lymphokine, Produkte der Zellen der natürlichen Immunabwehr, so auch TNF, wie Interferone, Interleukin-2, usw. in der Klinik und in der Krebstherapie einzusetzen und für diese neuen Einzelprodukte Indikationen zu erarbeiten. Bisher ist die Enttäuschung groß. Kürzlich wurden die Ergebnisse einer Studie über TNF veröffentlicht. Bei Anwendung von TNF bei 400 Krebspatienten konnte nur in 5 bis 10 % der Fälle ein flüchtiger Effekt mit starken Nebenwirkungen festgestellt werden.

Aus den obigen Ausführungen wird auch klar, warum diese Bemühungen von Anfang an zum Scheitern verurteilt sind. Die meisten gentechnologisch herstellbaren Lymphokine, wie TNF, Interleukin-2, Interferone, sind nichts anderes als Boten- und Verstärkersubstanzen der natürlichen Immunabwehr. Es liegt somit auf der Hand, daß zunächst für jeden einzelnen Krebspatienten in autologen Testsystemen ermittelt werden sollte, ob überhaupt eine Abwehrreaktion vorliegt, nämlich eine tumorassoziierte Immunreaktion. Wenn sie vorliegt, ist festzustellen, in welcher Qualität und welcher Stärke und auf welcher Ebene der Kaskade des komplizierten Immunabwehrsystems etwas vorliegt. Erst nach der Feststellung und Vorliegen dieser Daten sollte dann eine geeignete stimulierende Substanz oder Stoffkombination ausgesucht und gezielt therapeutisch eingesetzt werden. Die notwendigen Techniken und Erkenntnisse sind jetzt bekannt. Es ist somit nur eine Frage der Ressourcen und des Willens in diesem Bereich der Immuntherapie des Krebses gezielt und mit Erfolg vorzugehen.

Dies sei noch einmal an dem oben erwähnten Beispiel Arabinogalaktan/TNF erläutert:

Hier werden zwei therapeutische Prinzipien miteinander konkurieren, nämlich die Substitutionstherapie und die Induktionstherapie. Der Preis für Echincea-Extrakt liegt wesentlich niedriger als der für biotechnologisch hergestelltes TNF. Dennoch wird Echinacea im Körper des Patienten in vielen Fällen mehr TNF freisetzen als bei der TNF-Substitutionstherapie wirtschaftlich realisiert werden kann. Durch Impfen/Vakzinieren von Tumorpatienten mit autologen Tumorzellen/Tumormaterial hat man weltweit in mehreren Arbeitsgruppen beim Dickdarmkarzinom (colorectal carcinom) und Mammakarzinom (breast cancer) gute klinische Langzeitresultate erzielt. Auch hier wird man noch bessere Resultate erzielen können, wenn man die oben genannten Ausführungen mitberücksichtigt und nur bei solchen Patienten vakziniert, bei denen überhaupt eine tumorassoziierte Immunreaktion meßbar ist. Weiterhin wird man beim Vakzinieren es kontrolliert tun mit der Absicht, eine effektive Abwehrreaktion gegen die Krebszellen zu induzieren und aufrechtzuerhalten. Es wird somit möglichst eine antikörpergesteuerte zelluläre Immunreaktion mit komplementabhängigen tumorassoziierten IgG-Antikörpern angestrebt.

Ein typisches Beispiel für das erfindungsgemäß entwickelte Prinzip der kombinierten Krebstherapie mit kontrolliertem und reguliertem Immunsystem ergibt sich aus dem nachfolgend geschilderten Fall einer unheilbar krebskranken Patienten, bei der die schulmedizinische Standardtherapie inzwischen versagt hatte oder unwirksam geworden war. Es handelt sich um den Fall einer 58-jährigen Brustkrebspatientin, im Winter 1985/1986:

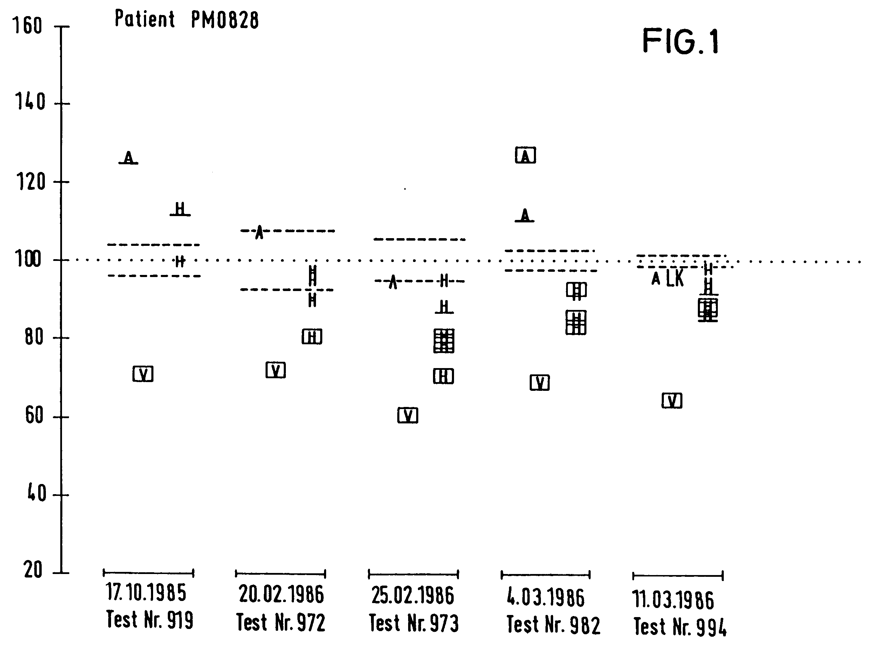

Am 17.10.1985 sucht die Patientin den ärztlichen Leiter des Serobac^{(R)} -Institutes, den Erfinder, auf, da trotz chefärztlicher Behandlung in einem anderen Krankenhaus die Krebskrankheit fortschreitet. Die Primärtherapie 1979 war pT2a, Primärtumordurchmesser 2,8 cm, 5 befallene Lymphknoten Biotyp B(CEA positiv). Damals über perioperative Alpha-2-Serumspiegelanalyse Annahme einer immunologischen Aktivität des Tumorgeschehens. Ablatio mammae. 6Cyclen Leukeran-Methotrexat-5-Fluorouracyl nach dem Protokoll der Düsseldorfer Mammakarzinomstudie. 9 Monate nach Primärtherapie Orbitametastase links Betatronbestrahlung. 15 Monate nach Primärtherapie Lungenmetastasierung mit Luftnot. Einsetzen einer kombinierten Tamoxifen-Clinovir-Therapie (3x10 mg Tamoxifen plus 3 x 100 mg Clinovir per os). Ursprünglich lag ein rezeptorpositives Mammakarzinom vor: Estrogen- und Progesteronrezeptoren mittelstark positiv. Auf Hormontherapie Vollremission. Nach drei Jahren, nach dem klinisch röntgenologisch o.B. (CT-Thorax,-Kopf,-Abdomen und Knochenszintigramm), Absetzen der Hormontherapie durch andere Ärzte. Nach weiteren 1 1/4 Jahren, im Herbst 1984, erscheinen von Hautknötchen/Hautmetastasen. Februar 1985 positives Knochenszintigramm. Wiederverordnen von Tamoxifen. Bis Oktober 1985 Weiterwachsen der Hautmetastasen (ca. 20 bis 25), größte am Kopf, im linken Halsbereich bis Walnußgröße. Versuch der hochdosierten MPA-Therapie. Vorübergehende Abflachung des CEA-Serumspiegelanstieges. Am 15. Oktober 1985 Entfernung von zwei kirschgroßen Hautmetastasen aus dem Rücken, pravertebral Hormonrezeptorstatus positiv für Estrogen- und Progesteronrezeptoren. Verarbeitung der Tumorcytosol- und Tumorsedimentreste zum Antigen. Am 17. Oktober 1985 LMI-Test: positive tumorassoziierte Immunreaktion mit Migrations-Enhancement, wie beim klinisch manifest metastatisiertem Mammakarzinom üblich ist. Gute allgemeine Immunreaktion auf Recallantigene Streptokinase/Streptodornase (V-Zeichen in Abbildung 1). Ab 31. Dezember 1985 Beginn der systematischen Applikation, intravenös, des pflanzlichen Mistelgesamtextraktes (Visci albi herba/Mistelkraut) (englisch: mistletoe extract) auf ausdrücklichen Wunsch der Patientin, nachdem im Dezember 1985 alle schulmedizinischen therapeutischen Möglichkeiten ausgeschöpft und unwirksam geworden sind. Appliziert wurde das Handelspräparat Plenosol^{(R)} der Firma Dr. Madaus & Co., Köln. Beim Plenosol^{(R)} handelt es sich um ein Mistelgesamtextrakt. Die Einstellung der Stärke des Präparates erfolgt über Jahrzehnte über die sogenannten Nekroseeinheiten. Das Präparat bzw. der Pflanzenextrakt wird in Verdünnungsstufen in die Kaninchenhaut injiziert und hier an dieser Stelle entsteht eine Rötung, deren Durchmesser ausgemessen werden kann. Es handelt sich somit weniger um Hautnekroseeinheiten, sondern um die Quantifizierung einer lokalen, sterilen Entzündungsreaktion mit Hautrötung und Hautinduration, da es zur Hautnekrose nur in der höchsten Konzentration kommt.

Die Autoren Dinkelaker und Kass im Buch: "Die Mistel in der Therapie" (Haug-Verlag 1982), ISBN 3-7760-0655-2, empfehlen unter anderem die intravenöse Therapie bei Krebspatienten als eine unspezifische Reiztherapie im Sinne der antroprosophischen Betrachtungsweise, und zwar mit aufsteigenden Dosen bis zu 3 Monaten und dann die höchste Dosierung von 700 NE (Nekroseeinheiten), d.h. 0,7 ml der Ampulle Stärke I mit 1000 NE/ml. Beim Lungen- und Bronchialkarzinom geben die Autoren auf Seite 55 eine zu erreichende Enddosis mit 0,3-0,-5-bis 1,0 ml Plenosol^{(R)} Stärke II an. Schulmediziner lehnen die Verwendung von Mistelextrakten als Medikament der Krebstherapie ab. Dies ist auch in gerichtlichen Streitverfahren belegt worden, wo es um die Behandlungskosten der Misteltherapie ging.

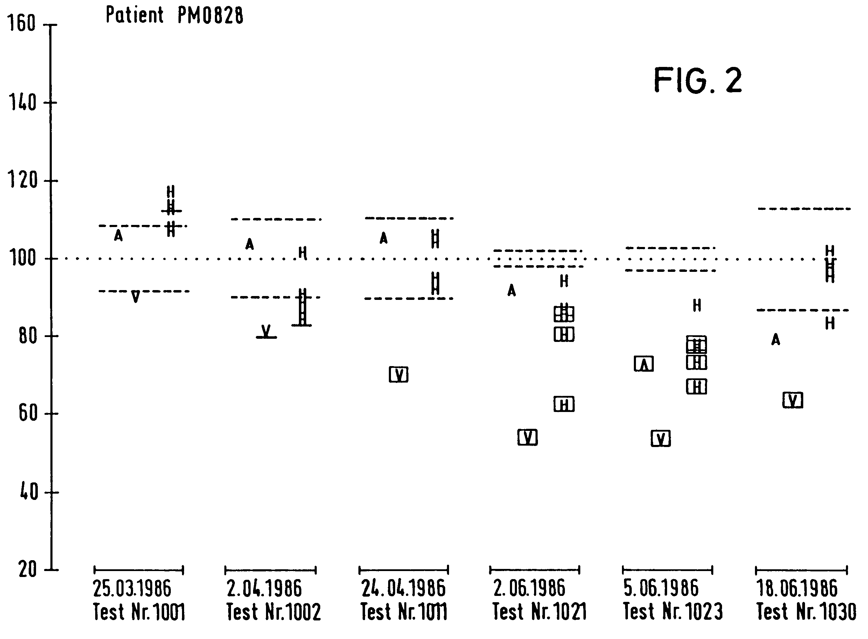

Bei der oben genannten Patientin wurde ab 31. Dezember 1985 das Mistelpräparat Plenosol^{(R)} in der Stärke II pro Ampulle pro Woche appliziert, d.h. 20 000 NE pro Woche intravenös. Dabei wurde die tumorassoziierte Immunreaktion im LMI-Test sowie die Gesamt-Lymphozytenzahl bestimmt. Die Gesamt-T-Zell-Zahl und die funktionellen Subgruppen der Lymphozyten, Helfer-Zellen, Supressor-Zellen und Natural-Killer-Zellen (NK-Zellen) wurden fortlaufend bestimmt. Der Effekt auf das Tumorgeschehen wurde über den Serum-CEA-Tumormarkerspiegel dokumentiert und konnte anhand des Verhaltens der Hautmetastasen beobachtet werden (siehe Abbildungen 1 und 2).

Am 20. Februar 1986 erfolgte Kontrolle der tumorassoziierten Immunreaktion, nachdem es der Patienten subjektiv etwas besser ging. Der Serum-CEA-Spiegel stieg trotzdem kontinuierlich an. Es trat ein verblüffender Effekt der Misteltherapie beim LMI-Test ein. Ab 20. Februar 1986 wurde das Mistelpräparat höherdosiert, um den therapeutischen Effekt ausreizen zu können. Es wurden täglich eine Ampulle Plenosol^{(R)} der Stärke II i.v. appliziert. Die LMI-Testkontrolle ergab Zunahme der Migrationsinhibition, d.h. Durchbrechung der Immuntoleranz und der Supressoraktivitäten. Erscheinen von leichter Rötung um die Hautmetastasen herum. Computerzeichen A = Reaktion gegen 20-mü-Tumorgewebegefrierschnitt als Antigenquelle. Punktscharen H = verschiedene Antigendosen aus präpariertem Tumorcytosolantigen und Tumorsedimentantigen. Einmalunterstrichen = einfach signifikante Reaktion gegenüber Kontrollansatz, jeweils dreimal angesetzt. Kästchen = zweifachsignifikante Reaktionen. Ab 25. Februar 1986 bis zum 11. März 1986 Applikation von 3 x 1 Ampulle Plenosol^{(R)} Stärke II i.V. pro Woche und danach ab 11. März 1986 nur 1 x 1 Ampulle Plenosol^{(R)} Stärke II i.v. pro Woche. LMI-Testansatz gegen Varidase (= Streptokinase und Streptodornase) als Recallantigene zum Messen der allgemeinen Immunität und zur Systemkontrolle. Die Erhöhung der Misteltherapie führte zu einer Rötung um die Hautmetastasen, die an Stärke zunahmen, während Serum-CEA und Serum-CA-15-3-Anstieg abflachte und stationär wurde. Ab 11. März 1986 Rückgang auf die Dosierung vom 31. Dez. 1985, nachdem sich ein negativer Effekt abzeichnete, nämlich im LMI-Test und in einer Schädigung der Gesamt-T-Zellenzahl, der Helferzellzahl und partiell auch der Supressorzellzahl (siehe Tabelle 1). Am 25. März 1986 Vorhalten und Zunahme des negativen-toxischen Effektes mit Zusammenbruch der allgemeinen Immunität (V-Zeichen), Hauttest Multitest^{(R)}-Institut Meriuex mit pathologischem Score (sieben verschiedene Bakterien- und Pilzantigene), im LMI-Test erneut Migrations-Enhancement/Immuntoleranz mit Verschwinden der Hautrötung um die Hautmetastasen, Tumormarkerserumspiegel (CEA + Ca-15-3) erneut steigend. Es wurde jetzt versucht, das Mistelpräparat mit einem Thymuspräparat zu kombinieren. Dies führte zu einer optimalen mitogenen Immunstimulation. Es erfolgte der Aufbau einer starken tumorassoziierten Immunreaktion im LMI-Test mit Migrationsinhibition gegen alle Tumorantigendosen und auch klassisch mit Tumorgewebeschnitt als Antigenquelle. Starke Rötung um die Hautmetastasen herum, Verschwinden von linsengroßen Hautmetastasen und zum Teil auch von größeren Tumorknoten bis Bohnengröße. Walnußgroße Knoten wurden meßbar etwas kleiner. Rückgang der Tumormarkerserumspiegel 5 bis 7 % pro Woche. Im Knochenszintigramm Teilremission. Der Patientin ging es jetzt subjektiv gut. Sie ging wieder ihrer Arbeit als selbständige Papierwarenhändlerin nach, obwohl zu diesem Zeitpunkt 150 bis 250 g Tumorgewebe im Körper vorhanden war. Bis jetzt keine nennenswerten Nebenwirkungen der Gesamttherapie. Appliziert wurde 1 Ampulle Plenosol^{(R)} Stärke II pro Woche (20 000 NKE) i.v. und 3 x 2 Ampullen Thymovocal^{(R)} intramusculär. Es handelte sich um ein Gesamtthymusextract der Firma Dr. Mulli Nachfolger GmbH & Co. KG, Neuenburg (ca. 22 mg Peptidgemisch). 2. Juni bis 5. Juni 1986: Erhöhung der Medikamentendosierung auf täglich 1 x 1 Ampulle Plenosol^{(R)} Stärke II bei Beibehaltung der Dosierung von Thymuvocal^{(R)}. Hierauf zusätzliche Verstärkung der tumorassoziierten Immunreaktionen klinisch und auch ex-vivo-in-vitro. 5. Juni bis 18. Juni 1986: Weglassen des Mistelpräparates aus der Medikamentenkombination bei gleichzeitiger Weitergabe des Thymuspräparates Thymuvocal^{(R)} 3 x 2 Ampullen pro Woche i.m.. Promptes Nachlassen der tumorassoziierten Immunreaktionen, klinisch und ex-vivo-in-vitro, die im LMI-Test meßbar war und nachweisbar ist. Klinisch Rückgang und Verschwinden der Rötung um die Hautmetastasen herum. Erneut steigender Tumormarker-Serumspiegel und Wiedererscheinen der verschwundenen linsengroßen Hautmetastasenknötchen, Größenzunahme der größeren Tumorknoten.

Aus diesem und einer Reihe weiterer inzwischen untersuchter Fälle gelangt der Erfinder zu folgenden neuen Erkenntnissen: In pflanzlichen Extrakten, aber auch in einigen tierischen Extrakten, wie in Schlangengiften, müssen immunpharmakologisch hochwirksame Substanzen vorhanden sein, die eine tumorassoziierte Immunreaktion in der Weise verstärken, daß sie die entsprechenden funktionellen Subgruppen der Lymphozyten aktivieren, und zwar vorwiegend die zytotoxisch aktiven Lymphozytensubpopulationen. Dabei ist dieser Aktivierungsprozeß in der Lage, eine supressorsubstanzen- und supressorzellen-bewirkte Immuntoleranz zu durchbrechen (nachweisbar im LMI-Test oder im LAI-Test ex-vivo-in vitro) und dabei eine zellvermittelte und eine antikörperabhängige zellvermittelte Zytotoxizität zu erneuern, und dies trotz Antigenpersistenz und dabei die Abwehrreaktion zusätzlich im erheblichen Umfang zu verstärken. Die bisher untersuchten immunpharmakologischen Wirkstoffe gehören fast ausnahmslos in die Gruppe der Polysaccharide und der Lektine. Lektine sind Glykoproteine pflanzlicher oder tierischer Herkunft mit Zuckerspezifitäten. Sie haben als größere Moleküle die funktionelle Eigenschaft, ähnlich wie Antikörper, nach dem Türschloß/Schlüssel-Prinzip ihre Rezeptoren bzw. Liganden spezifisch zu erkennen, so wie Antikörper die zugehörigen Antigene. Demnach scheinen diese Lektine eine wichtige biologische Signalübertragungsfunktion auszuüben. Stoffe, die in-vitro in der Lage sind an geeigneten Zielzellen, wie menschlichen Lymphozyten, DNS-Neusynthese und damit H3-Thymidin-Einbau auszulösen (gegebenenfalls auch eine Zellteilung auszulösen) werden als mitogene Substanzen bezeichnet. Klassische Vertreter dieser Substanzen sind die sogenannten mitogenen Lektine, wie z.B. Concanavalin A (Con A) und Phythämagglutinin (PHA). Diese Substanzen werden bisher bei in-vitro-immunologischen Untersuchungen eingesetzt, um die Stimulierbarkeit der Abwehrzellen von Patienten in verschiedenen Krankheitszuständen oder die von Versuchstieren überprüfen zu können. Darüber hinaus fanden mitogene Lektine Anwendung in buffy-coat von Blutspendern (isolierte Abwehrzellen insgesamt, einschließlich der Leukozyten) oder in Kulturen von lymphatischen Dauerzell-Linien, um Lymphokine, wie Interferone und Interleukin-2, induzieren und biotechnologisch herstellen zu können. Diese mitogenen Lektine fanden jedoch wegen der extrem hohen Toxizität bisher am Patienten keine therapeutische Anwendung. Andererseits fand der Erfinder, daß durch Entfernung der Mistellektine (Mistellektin I + II + III) aus dem Gesamtmistelextrakt die immunpharmakologische Wirkung des Präparates Plenosol^{(R)} (Gesamtmistelextrakt) fast vollständig verlorengeht.

Kombiniert man hingegen den Gesamtmistelextrakt mit thymomimetischen Substanzen, so ist es möglich, die Immuntoleranz zu durchbrechen und ein Migrations-Enhancement in eine Migrationsinhibition im LMI-Test umzuwandeln. Als thymomimetische Substanzen wurden erfolgreich erprobt: Thymuvocal^{(R)} i.m. 3 x 2 Ampullen pro Woche, oder noch wirksamer, zwei- bis dreimal 1 Ampulle Timunox^{(R)} 100 subcutan mit je 50 mg TP5. In 9 Fällen von Mammakarzinompatientinnen und 3 Ovarialkarzinompatientinnen war diese Therapie bereits erfolgreich, d.h. in 12 von 12 Fällen. In jedem dieser Fälle wurde die immunbiologische ex-vivo-in-vitro-Eingangs- und Kontrolluntersuchung unter Verwendung von autologen Tumormaterial als Antigenquelle (vergl. WO 88/09935) durchgeführt. In allen 12 Fällen konnte klinisch eine positive therapeutische Wirkung auch anhand der Tumorsymptome beobachtet werden. Demgegenüber wurde in 4 von 4 anderen Fällen im LMI-Test keine nennenswerte Wirkung mit lektinfreiem Mistelextrakt beobachtet. Auch klinisch wurde keine Besserung beobachtet. Die therapeutische Wirkung besteht somit in der oben beschriebenen Kombination der immunpharmakologischen Wirkung und Effektivität der Mistellektine sowie durch die zusätzliche Applikation thymomimetischer Substanzen. In-vitro-Versuche haben dies bestätigt, indem sowohl Gesamtmistelextrakt, wie die isolierten Mistellektingemische dosisabhängig eine mitogene Aktivität auslösen, sie stimulieren den Einbau von H3-Thymidin in normale oder auch stimulierte Lymphozyten und bewirken sogar oftmals eine Lymphokinproduktion. Dies kann in-vitro über die Gesamtlymphokinantwort und über die Beeinflussung der Migration von isolierten Leukozyten (buffy-coat) festgestellt und belegt werden.

Die Mistellektine I, II und III haben die Spezifitäten Galactose, Galaktosid, Lactose und Mellobiose. Dies kann durch einfache Absorptionschromatographie, z.B. durch die bekannte Kopplung dieser Zucker an geeignete Sepharosepräparationen, festgestellt werden, die aus dem Gesamtmistelextrakt entsprechende Lektine entfernen. Die Therapie mit Mistelgesamtextrakt ist somit als mitogene Immunstimulation anzusprechen, die auch in-vivo therapeutisch wirksam ist. Dabei ist jedoch zu beachten, daß diese Wirkstoffe lymphozytenverbrauchend sind. Erfindungsgemäß wird dies kompensiert durch die zusätzliche Applikation thymomimetischer Substanzen.

Die bekannte und empfohlene, intravenöse Plenosol^{(R)}-Monotherapie geht nur bis zu einer Dosierung von maximal 700 Nekroseeinheiten pro Applikation. Setzt man Gesamtmistelextrakt klinisch-therapeutisch in dieser Dosierung ein, so kann man aber die zelluläre antikörpergesteuerte Immunreaktion nur unwesentlich verstärken. Bei dieser Dosierung ist es auch nicht möglich, die Immuntoleranz/Enhancement in Inhibition zu konvertieren und damit einen klinisch meßbaren therapeutischen Effekt zu erzielen.

Wird hingegen die Therapie mit Gesamtmistelextrakt über die empfohlenen Mengen hinaus erhöht, kommt man zwar zu stärkeren immunpharmakologischen Effekten, diese sind jedoch ausgeprägt lymphozytenverbrauchend. Es reicht in diesem Fall nicht aus, ein nur schwach wirksames Thymuspräparat in der empfohlenen Menge anzuwenden. Es ist vielmehr notwendig, entweder höher zu dosieren oder ein höherwirksames thymomimetisches Mittel einzusetzen. Außer dem bereits erwähnten Gesamtthymuspräparat Thymuvocal^{(R)} kommt daher auch ein vollsynthetisches Thymuspeptid, wie das Präparat Timunox^{(R)}100 der Firma CILAG in Frage, welches 50 mg des Wirkstoffes TP5 enthält.

Weitere geeignete thymomimetische Substanzen sind die Tetra- und Tripeptide TP4 und TP3 der Firma Richter Gedeon, Budapest. Hier handelt es sich um noch kürzere Peptidteile des Thymushormons, bestehend aus 38 Aminosäuren, aus denen bereits TP5 abgeleitet wurde. Als besonders wirksam hat sich die Kombination von 66,7 mg TP4 und 33,3 mg TP3 pro Ampulle/pro Applikation subcutan zwei- bis dreimal pro Woche erwiesen, und zwar in Kombination mit dem Gesamtmistelextrakt (Plenosol^{(R)}). Dazu wird auch der Gesamtmistelextrakt höherdosiert als bisher vom Hersteller empfohlen. Dabei sind stets die oben beschriebenen Kontrollen des Immunsystems durchzuführen.

Als weitere thymomimetische Substanz könnte sich die in der Erprobung befindliche Substanz Thymosin-Alpha-1 der Firma Hoffmann-La-Roche, Basel, erweisen, ein vollsynthetisches Thymushormon bestehend aus der gesamten Kette von 28 Aminosäuren.

Im Rahmen der obigen Untersuchungen wurde eine weitere interessante Beobachtung gemacht: Während der mitogenen Immunstimulation bei Krebspatienten (realisiert mit dem Mistelgesamtextrakt Plenosol^{(R)} sowie der thymomimetischen Substanzen, wie Thymuvocal^{(R)} und Timunox^{(R)}100), wurde regelmäßig reproduzierbar eine mäßige aber hochsignifikante (p kleiner als 0,001) Vermehrung der eosinophilen Granulozyten im peripheren Blutausstrich beobachtet. Vor dem Therapiebeginn beträgt der prozentuale Anteil der Eosinophilen 1,7 % (N = 23 Patienten) und in der 6. Woche der Immuntherapie durchschnittlich 5,9 %. Es ist bekannt, daß beim klinischen Einsatz von gentechnologisch hergestellten Interleukin-2, hochdosiert, beim Einsatz eines Schlüssel-Lymphokins der T-Zellen im Rahmen der clonalen Expansion und der Aktivierung, ebenfalls regelmäßig und hochsignifikant Vermehrung der eosinophilen Leukozyten im peripheren Blutausstrich beobachtet wird, und zwar 10 bis 18 %. Es bestand somit die Vermutung, daß im Rahmen der mitogenen Immunstimulation es ebenfalls zur Freisetzung und Mehrproduktion von Interleukin-2 kommt, und zwar als autokriner Prozeß oder zumindest als Vermehrung der Interleukin-2-Rezeptordichte an den aktivierten Lymphozyten. Es wurden daraufhin von der oben beschriebenen Patientin Serumproben, die bei -70°C eingelagert waren, auf Tumornekrosefaktor- und Interleukin-2-Rezeptor-Serumspiegelkonzentration untersucht. Tatsächlich ging der Interleukin-2-Rezeptor-Serumspiegelkonzentration parallel mit dem klinischen Bild und der ex-vivo-in-vitro-meßbaren Reaktionen im LMI-Test. Dies ist ein Hinweis dafür, daß durch die klinische Anwendung der erfindungsgemäßen Stoffkombination bzw. kombinierten Anwendung der Wirkstoffe eine immunpharmakologische Verstärkung spezifischer immunologischer Reaktionen und Abwehrmechanismen stattfindet. Die Induktion der Interleukin-2-Rezeptordichte/Serumspiegelkonzentration ist inzwischen auch in anderen Behandlungsfällen realisierbar und nachweisbar. Offensichtlich wird der TNF-Serumspiegelanstieg, die Induktion der TNF-Produktion, nicht nur durch die Wirkstoffe des Gesamtmistelextraktes induziert, sondern hängt dieses bisher noch nicht beobachtete Phänomen damit zusammen, daß Reste und Antigene der absterbenden Tumorzellen durch Makrophagen abgeräumt werden. Bei der oben erwähnten Patientin waren zum Zeitpunkt der Immuntherapie im Organismus ca. 200 bis 300 g Tumormasse vorhanden. Die Induktion der TNF-Produktion ist somit kein Charakteristikum des hier beschriebenen Immuntherapieprinzips. TNF-Serumspiegelanstiege wurden bei einigen Patienten beobachtet, bei anderen nicht.

Aus Arbeiten von Rosenberg und Mitarbeiter in Washington geht hervor, daß dort periphere oder bevorzugt tumorinfiltrierende Lymphozyten unter Einwirkung von Interleukin-2 in-vitro in Zellkulturen vermehrt und weitgehend in sogenannte lymphokinaktivierte Killerzellen (LAK) umgewandelt werden, die dann 100-grammweise dem Patienten reinfundiert werden, ergänzt mit der weiteren Gabe von niedrigdosiertem Interleukin-2. Es spricht einiges dafür, daß die mitogene Immunstimulation gemäß vorliegender Erfindung einen ähnlichen Prozeß in-vivo auslöst, jedoch mit weniger Aufwand, jedoch mit der gleichen klinischen Effektivität.

Es spricht einiges dafür, zur weiteren Unterstützung der Therapie zusätzlich Interleukin-2 zu applizieren, und zwar niedrig dosiert, da es den erfindungsgemäßen Effekt schneller auslöst und somit wie ein Anlasser wirkt.

Gegenstand der vorliegenden Erfindung sind somit zunächst einmal Kombinationspräparate zur Tumortherapie mit kontrolliertem und reguliertem Immunsystem enthaltend
a) mitogen immunstimulierende Substanzen und
b) thymomimetische Substanzen.

In vielen Fällen wird man jedoch nicht auf feste Kombinationspräparate zurückgreifen, sondern eine Tumortherapie mit kontrolliertem und reguliertem Immunsystem dadurch durchführen, indem man wirksame Mengen
a) für mindestens einer mitogen immunstimulierenden Substanz und
b) mindestens einer thymomimetischen Substanz appliziert.

Ein weiterer Gegenstand der vorliegenden Erfindung ist somit auch die Verwendung von mitogen immunstimulierenden Substanzen zur Herstellung von Medikamenten zur kombinierten Anwendung derselben mit thymomimetischen Substanzen bei der Tumortherapie mit kontrolliertem und regulierten Immunsystem.

Vorzugsweise werden als mitogen immunstimulierende Substanzen Lektine oder lektinhaltige Extrakte eingesetzt. Diese Extrakte können aus Pflanzen oder Tieren stammen. Vorzugsweise zum Einsatz kommen können auch die bisher als toxisch angesehenen Lektine. Eine bereits vorhandene und für die Praxis brauchbare Darreichungsform stellt der lektinhaltige Mistelgesamtextrakt dar.

Als thymomimetische Substanzen kommen Thymusextrakte, synthetische Thymuspeptide oder thymomimetisch aktive Peptidfragmente in Frage.

Stets werden die beiden Komponenten so dosiert, daß es zu einer deutlichen Wirkung kommt. Dies wird durch die Untersuchung der üblichen Parameter im Serum kontrolliert.

Schließlich ist Gegenstand der vorliegenden Erfindung ein Verfahren zur verbesserten Kontrolle des Immunsystems vor und während der Tumortherapie, das dadurch gekennzeichnet ist, daß beim Leukozytenmigrationsinhibitionstest (LMI) und/oder beim Leukozytenadhärenzinhibitionstest (LAI) mit mehreren verschieden großen Antigendosen getestet wird, umd die T-zellabhängige, adoptive, tumorassoziierte Immunität ex-vivo-in-vitro zu messen.

Wird zum Beispiel das mitogene Lektin des Schlangengiftes verwendet, welches β-Galaktose-spezifisch und Calcium-Ionen-abhängig ist, so zeigt es eine sehr starke T-Lymphozytenspezifische mitogene Wirkung in-vitro und im Tierversuch, und zwar stärker als PHA, dem Standard-Stimulator-Lektin für T-Zellen. Setzt man dieses Lektin im Tierversuch ein, beispielsweise im Kaninchen, und mißt man die Stärke der T-zellabhängigen, antikörpervermittelten, zellulären Immunreaktion gegen Varidase (Streptokinase/Streptodornase) im LMI-Test, gegen ubiquitär vorkommende bakterielle Stoffwechselprodukte, so bekommt man die typische Reaktionsform der Verstärkung der Immunantwort im LMI-Test, d.h. die starke Zunahme der Migrationsinhibition. Auch dieses Lektin zeigt somit im Tierversuch, ähnlich wie Mistelgesamtextrakt beim Menschen, eine abwehrzellenverbrauchende Eigenschaft im Rahmen der Verstärkung der Immunantwort/Immunabwehr, so daß auch bei späterem klinischen Einsatz dieses mitogenen Induktors eine geeignete thymomimetische Substanz in Kombination miteingesetzt werden muß, um die iatrogene Lymphopaenie kompensieren zu können und eine klinisch-therapeutische Immunantwortverstärkung realisieren zu können. Dabei ist nochmals darauf hinzuweisen, daß weder mit dem niedrigdosierten Mistelgesamtextrakt noch mit den isolierten Wirkstoffen Xyloglucan und Arabinogalaktan sich ein immuntherapeutisches Konzept realisieren läßt. Beide Wirkstoffe wirken selektiv nur auf Granolozyten und Makrophagen und beeinflussen nicht meßbar die tumorassoziierte Immunreaktion im LMI-Test ex-vivo-in-vitro und zeigen daher keinerlei klinischen Effekt auf das Krebsgeschehen, wenn die Krankheit manifest wurde. Es ist somit nötig, den Gesamtmistelextrakt so hoch zu dosieren, daß er wirklich mitogen immunstimulierend wirkt.

Die intravenöse Applikation des Gesamtmistelextraktes / Plenosol^{(R)} oder eines ähnlichen Wirkstoffes/Wirkstoffgemisches ist zwar die bevorzugte Applikationsform, aber die gleichen Effekte (wenn auch systematisch in schwächerer Form und mehr lokofokal im Lymphabflußbereich der Applikationsstelle stärker wirkend), können festgestellt werden, wenn die Wirkstoffe subcutan oder intramuskulär appliziert werden. Für eine thymomimetische Substanz, wie z.B. Timunox^{(R)}, ist der bevorzugte Applikationsweg subcutan. Eine intravenöse Verabreichung ist ebenfalls bekannt und gängig.

Gesamtmistelextrakt/Plenosol^{(R)} läßt sich galenisch zusammen mit dem Wirkstoff des Präparates Timunox^{(R)}, mit dem Pentapeptid TP5, in der gleichen Ampulle unterbringen. Trotzdem wird man diese Medikamentenkombination meist getrennt und/oder in einer Kombipackung für die neue Indikation auf den Markt bringen und bewerben. Das Unterbringen der Wirkstoffe/Wirkstoffkombinationen im Sinne dieser Erfindung in einer Ampulle mit fest vorgegebenen Dosen/Wirkstoffverhältnissen würde eine individualisiert angepasste Immunantwortverstärkung für die Praxis schwieriger machen.

Bekannte Mistelpräparate im deutschsprachigen Raum sind: Iscador^{(R)}, Plenosol^{(R)}, Abnoba-Viscum^{(R)}, Helixor^{(R)}, Isorel^{(R)}. Bekannte Thymuspräparate, thymomimetisch wirksame Präparate sind beispielsweise im deutschsprachigen Raum: Thymuvocal^{(R)}, Gesamt-Thymuspräparat des Deutschen Thymusvereins e.V., TP 1^{(R)}, Timunox^{(R)}, um nur die wesentlichsten und effektivsten zu nennen, die parenteral applizierbar angeboten werden.

Thymuspräparate, wie Dragees, Kapseln, Zäpfchen, die für die perorale Therapie angeboten werden, sind meist Gesamtthymusextrakte und entfalten meist nur eine sehr schwache thymomimetische Wirkung in-vivo. Sie können daher für die Realisierung der beschriebenen Stoffkombinationen und für die Realisierung des therapeutischen Prinzips nicht bevorzugt empfohlen und herangezogen werden.

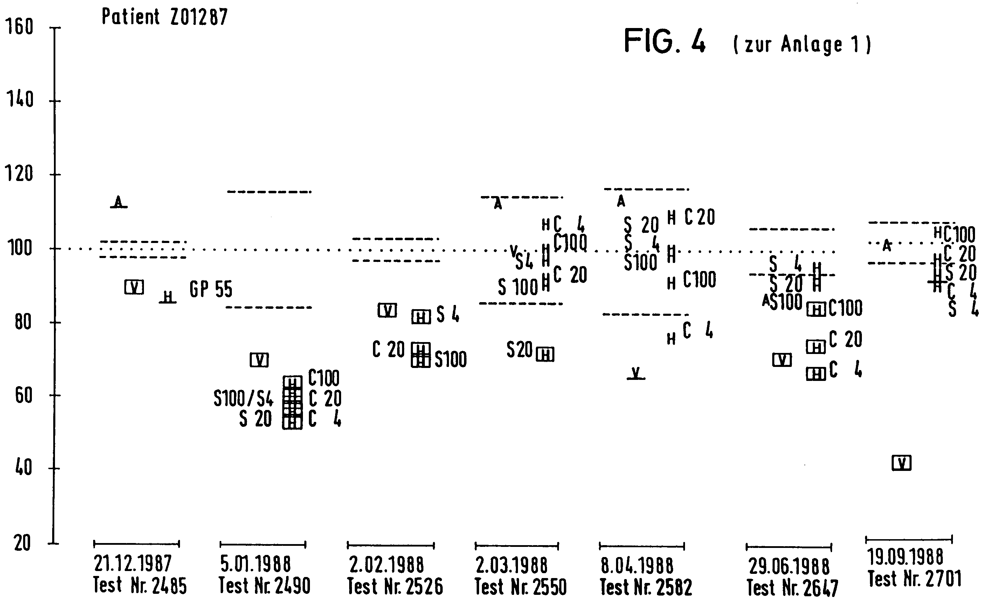

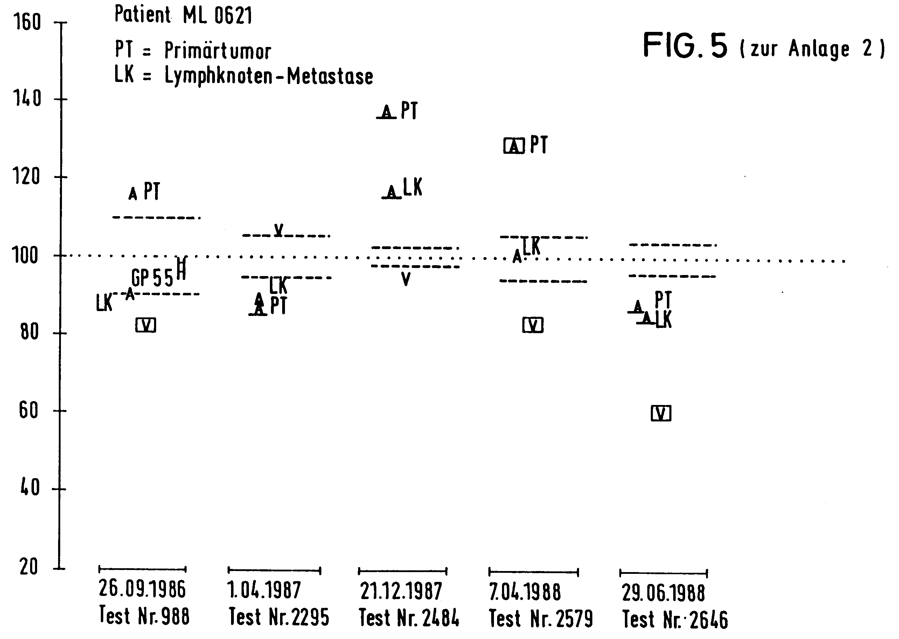

Die Abbildungen 1 bis 6, Tabelle 1 sowie die zugehörigen Beschreibungen (Anlagen 1 bis 3) schildern in detaillierter Form den Krankheits- und Therapieverlauf der oben erwähnten Patientinnen.

In der Zwischenzeit ergab ein intensives Literaturstudium der Arbeit von H. Franz: Inhaltsstoffe der Mistel (Viscum album L.) als potentielle Arzneimittel, Die Pharmazie 40(1985)97-104. Diese enthält einen zusammenfassenden Überblick mit Stand 1985 zum Wissensstand der Wirkstoffe im Mistelextrakt und zur Wirkungsweise dieser Inhaltsstoffe.

Die späteren diesbezüglichen Arbeiten, wie von T.A. Khwaja et al.: Recent Studies on the Anticancer Activities of Mistleio (Viscum album) and its Alkaloids, Oncology, 43: suppl. 1, pages 42-50(1986), bringen keinen substantiellen Fortschritt. Zwar werden in dieser und in den zitierten Arbeiten der gleichen Autoren in Tierversuchen die Wirksamkeit der Mistelextrakte und Bestandteile des Mistelextraktes zur Krebstherapie excellent untermauert, einschließlich der Tatsache, daß in Handelspräparaten deutschsprachiger Länder die wirksamen Bestandteile um den Faktor 10 bis 1000 schwanken. Aber die Autoren erkennen nicht das Wirkprinzip, daß in-vivo nicht die direkte tumorzellhemmende/tumorzellabtötende Wirkung der Bestandteile effektiv ist, da die hierzu notwendigen Wirkstoffkonzentrationen in-vivo kaum erreicht werden. Die Effektivität beruht allein auf immunologischer Grundlage.

Die Arbeit aus 1986 von T. Hajto et al.: Natural Killer and Antibody-Dependent Cell-Mediated Cytotoxity Activities and Large Granular Lymphocyte Frequencies in Viscum album-Treated Breast Cancer Patients, Oncology, 43: 93-97(1986 beinhaltet eine Arbeit mit altem Stand des Wissens zur immunologischen Wirkungsweise der Mistelextrakte, speziell hier mit Iscador. Iscador ist in Deutschland das meistverkaufte Mistelpräparat, aber - laut Untersuchungen von T. H. Khwaja - mit dem geringsten Lektingehalt pro mg Frischpflanzengewicht. Durch das Herstellungsverfahren (Fermentation) werden die Lektinwirkstoffe weitgehendst zerstört und in ihrer biologischen Wirksamkeit geschädigt.

Nach dem Anmeldetag der vorliegenden Erfindung (Priorität vom 01.06.1989) erschien die Arbeit von T. Hajto, K. Hostanska und H.J. Gabius: Modulatory Potency of the β-Galactoside-specific Lectin from Mistletoe Extract (Iscador) on the Host Defense System in-Vivo in Rabbits and Patients in Cancer-Research 49, 4803-4808, am 1. September 1989. Diese Arbeit bestätigt die Erkenntnisse der vorliegenden Erfindung, daß die immunpharmakologisch relevanten Wirkstoffe der Mistelextrakte/Mistelpräparate die Mistellektine und vor allem das Mistellektin I sind/bzw. ist. Trotzdem erkennen die Autoren den Zusammenhang nicht, daß die Mistelextrakt/Mistellektin-Therapie, wenn sie immunpharmakologisch in adäquater Dosis gegeben wird, lymphozytenverbrauchend ist und unabhängig hiervon zur vollen Entfaltung einer klinisch wirksamen Immuntherapie: mitogene Immunstimulation führt, wobei die zusätzliche Gabe einer geeigneten thymomiteischen Substanz notwendig ist.

Auch die Wirkung von Interleukin-2 ist durch weitere Ergebnisse bestätigt worden. Die systematischen Untersuchungen ergaben eindeutig, daß die immunpharmakologische Hauptwirkung der mitogenen Immunstimulation aus dem additiven Effekt der Lektininduktoren und der thymomimetischen Substanz(en) besteht, in der Weise, daß
erstens die thymomimetische Substanz (TP5 und/oder TP4/TP3)
a) die Gesamtlymphozytenzahl und die Gesamt-T-Zellzahl anhebt
b) die Helfer/Supressorzellen-Ratio zugunsten der Helferzellen verbessert
c) die Lymphozyten aktiviert (= Erhöhung der Zahl der HLA-DR-positiven und/oder der Transferrinrezeptorenpositiven Lymphozyten)
und
zweitens die Lektin-Induktoren hochsignifikant erhöhen
a) die Zahl der Interleukin-2-Rezeptoren-positiven Lymphozyten durchgehend unter mehreren funktionellen Subgruppen der Lymphozyten einschließlich der Natural-Killer-Zellen,
b) aber mit Schwerpunkt die Zahl der cytotoxischen Lymphozyten (= CTL),
c) wobei die Gesamtzahl der Natural-Killer-Zellen (=NK-Zellen) nicht wesentlich beeinflußt wird.

Die induzierte Verschiebung in der Zusammensetzung der funktionellen Subgruppen der Lymphozyten ist auch von der Qualität der tumorassoziierten Immunantwort/Abwehrreaktion abhängig. Zwar läßt sich eine Aktivierung des NK-Zellensystems nachweisen, ähnlich gelagert wie dies von anderen Autoren beschrieben wird (T. Hajto et al. 1986 und T. Hajto et al. 1989), die Hauptdomäne/die Hauptwirkungsweise der mitogenen Immunstimulation ist jedoch eine exponentielle Verstärkung der Cytotoxischen-Lymphozyten-Funktionen (T-Zellabhängige zelluläre Zytotoxizität CTL) und der Antikörpergesteuerten-zellulären-Zytotoxizität (ADCC-Reaktion), mit Durchbrechen einer Immuntoleranz in der tumorassoziierten Immunantwort bei klinisch manifester Krebserkrankung. Dementsprechend stellt sich im Laufe der Behandlung jeweils ein typisches Bild der funktionellen Subgruppen der Lymphozyten ein, in Abhängigkeit von der Qualität der tumorassoziierten Immunantwort (siehe Tabelle 2).

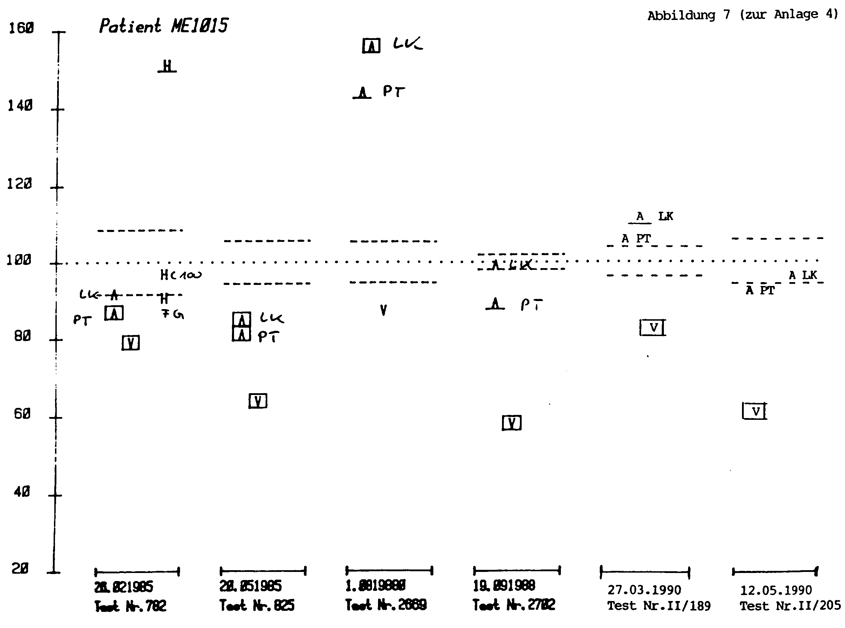

Bei klinisch manifester Krebserkrankung mit Tochtergeschwulsten ist durch die mitogene Immunstimulation erst dann ein meßbarer, belegbarer Heileffekt nachweisbar, wenn im autologen Testsystem im Leukozytenmigrationsinhibitionstest (LMI) in ihrer optimierten Form, ex-vivo-in-vitro eine positive, T-zellabhängige, adoptive, tumorassoziierte Immunreaktion gegeben ist. Je stärker und ausgeprägter diese ex-vivo-in-vitro meßbare tumorassoziierte Reaktion ist, desto höher ist das therapeutische Potential der mitogenen Immunstimulation. Diese Aussage belegt der weitere Krankheits- und Behandlungsverlauf der Mammakarzinompatientin ME 1015, beschrieben auf Anlage 3 und im weiteren Verlauf auf Anlage 4 mit Abbildung 7 (zur Anlage 4).

Dies schließt nicht aus, daß eine Mistelextrakttherapie und so auch die mitogene Immunstimulation auch in solchen Tumorbehandlungsfällen einen positiven, klinischen Therapieeffekt bewirkt, wo im LMI-Test keine Reaktion zu messen ist und eine tumorassoziierte Immunreaktion nur auf der Ebene der Makrophagen, NK-Zellen gegeben ist. Dieser positive klinische-therapeutische Effekt dürfte jedoch nur in postoperativen, adjuvanten Behandlungsfällen von pragmatischer Bedeutung sein, wo nur einzelne Tumorzellen und Mikrometastasenherde vorhanden sind. Ein Heileffekt dürfte nur durch adjuvante Therapiestudien zu beweisen sein und nicht anhand einzelner Behandlungsfälle.

### Mammakarzinompatientin ZO, A1287 Anlage 1 60 Jahre alt.

OP 21.12.1988 SS, Ablatio mammae li. PT3, PNo, Mo, G3 Primärtumordurchmesser über 5,0 cm. Lymphangiosis carcinomatosa im Tumorbett.

| | |
|---|---|
| Cytosol-CEA | 3,63 ng/mg Tum.prot. |
| Cytosol-Ca-15-3 | 57,32 ME/Tum.prot. |
| Cytosol-TPA | 1046,60 U/Tum.prot. = hochgradig positiv, schnell wachsend. |
| Estrogen-Rezeptoren | 98 femtomol/mg Tum.prot. |
| Progeteron-Rezeptoren | 75 femtomol/mg Tum.prot. |

3 Cyclen-GMF + 24 Monata Tamoxifen-Therapie nach dem BMFT-Mammakarzinom-Studie zwischen den Chemotherapiecyclen und 4 Wochen lang danach Timunox-Immunprotektion, Immunrestauration, Chemotherapiebeginn jedoch erst am 14. Tag postop.

21.12.1989 OP-Tag Enhancement, wie zu erwarten ist, mit der Gewebsschnittechnik bei guter Allgemeinimmunität (V). Gp-55 Kreuzreaktivität (Biotyp-A des Mammakarzinoms).

05.01.1988 am 14. postop. LMI mit hochgradiger Inhibition mit allen Cytosol- und Sediment-Antigen-Dosen, gute Recall-Immunantwort.
02.02.1988 1. Tag II. Chemotherapiecyclus.
02.03.1988 1. Tag III. Chemotherapiecyclus.

Zunehmendes Verschwinden, Auslöschen der hochgradigen tumorassoziierten Immunstatus/Immunantwort bereits durch 3 Cyclen CMF bei der 61-jährigen, postmenopausalen Patientin. Cave-Verlauf der allgemeinen (Recall)-Immunantwort trotz Timunox-Therapie.

08.04.1988 5 Wochen nach dem 1. Tag des letzten Chemotherapiecyclus. Bis zu diesem Zeitpunkt nur Immunrestauration mit Timunox, 100,2 bis 3 x 1 Ampulle subcutan. An diesem Tag Gesamt-T-Zellzahl 80% (normal von 58 - 75%) bei Gesamt-Lymphozytenzahl von 2100/cmm. Helfer-Zellen 59%, Supressorzellen 23%, NK-Zellen 15%, d. h. trotz der normalen Lymphozytensubpopulationen hochgradige Beeinträchtigung der tumorassoziierten Immunreaktion durch die Chemotherapie, durch 3. Cyclen-CMF und dies trotz der Timunox-Therapie.

20.04.1988 bis 29.06.1988 1. mitogene Immunstimulationskur. Hochgradige Verstärkung der tumorassoziierten Immunreaktion gegen lösbare/Cytosol-Antigenfraktion (möglicherweise gegen virale Genprodukte) jedoch keine meßbare tumorassoziierte Immunreaktion mehr gegen Sediment/Zellmebranantigenfraktion, obwohl postoperativ am 05.01.1988 die gleiche Antigenpräparation eingesetzt worden ist.

Vermerk: Dies ist eine typische Reaktion auf die Chemotherapie beim Biotyp-B des Mammakarzinoms, in der Postmenopause: Der größte Teil der Antigenität stammt aus dem Zellinneren. Die tumorassoziierte Immunreaktion gegen Zellwandantigene wird durch die Chemotherapie stärker, ausgeprägter geschädigt.

10.08.1988 bis 19.09.1988 2. mitogener Immunstimulationstherapiecyclus. Trotz der Immunverstärkung nur eine minimale, meßbare tumorassoziierte Immunreaktion (einfach signifikant nur) gegen die geringste Antigendosis (Cytosol 4 mikrogramm/ml), bei mächtigster Verstärkung der allgemeinen Immunreaktion gegen die eingesetzten Recallantigene. (V-Zeichen)

Vermerk: Weitere mitogene Immunstimulation ist nach dem heutigen Stand des Wissens nicht angezeigt. Aktiv-spezifische-Immunstimulation/Vakzination wäre bei dieser Pat. an dieser Stelle, angezeigt bei dem hohen Restrisiko.

### Patientin ML0621 Anlage 2 63-jährige Mammakarzinompatientin

### DEMONSTRATION DES VERGLEICHENDEN EFFEKTES/MONOTORING VON THYMUSMONOTHERAPIE (TIMUNOX) VERSUS MITOGENE IMMUNSTIMULATION INTRAINDIVIDUELL

Patientendaten bitte zum Teil aus dem Beiblatt entnehmen. Ergänzung:
Primärtherapie im 9/1985. Damals pT2, Primärtumordurchmesser 3,9 cm, 11 befallene axillären Lymphknoten. Estrogen- und Progesteronrezeptorkonzentration grenzwertig.

26.09.1985 erster LMI am 14. postop. Tag, daß heißt nach Applikation des 1. perioperativen adjuvanten Chemotherapiecyclus am 1. und 8. postop. Tag entsprechend dem BMFT-Mammakarzinom-Studien-Therapieprotokoll 2. Hiernach bei guter allgemeiner Immunität (V-Zeichen) keine meßbare tumorassoziierte Immunität mehr.

Vermerk: Cave Postervortrag der Arbeitsgruppe auf dem Jahreskongreß der Deutschen Krebsgesellschaft im 3/1986 in München. Dort konnte belegt werden, an ca. 40 Fällen, daß durch die Chemotherapie, bereits nach einem perioperativen Chemotherapiecyclus die tumorassoziierte, T-zellabhängige, meßbare Immunität, signifikant, hochgradig, beschädigt, negativ beeinflußt wird.

Nach dem IV. Chemotherapiecyclus Abbruch oder adj. Chemotherapie wegen Unverträglichkeit, Lympho-Leukopaenia, mit hoher Wahrscheinlichkeit auf dem Boden/Reaktivierung eines Virusinfektes.

01.04.1987 kurze stat. Durchuntersuchung. Hier meßbare tumorassoziierte Immunität gegen Primärtumorgewebe (Gefrierschnitttechnik) bei noch weiterhin sehr schlechter Allgemeinimmunität (V).

21.12.1987: Steigende Tumormarkerwerte Ca-15-3 und TPA. CA-15-3 pathologisch, über 32 mE/ml. CEA normal (der Tumor war Cytosol-CEA negativ, möglicherweise Biotyp-B, gp 55-Immunantwort konnte nicht getestet werden).
Es baute sich eine hochgradige, tumorassoziierte Immunreaktion auf, im Rahmen der beginnenden Progression, jedoch typischerweise, wie theoretisch und praktisch zu erwarten ist, mit Enhancement, alkalische Phosphatase, Gamma-GT, Lebersonogramm, röntgenologisch/klinisch o.B. (AP, G-GT, Rö-Thorax, Knochenszintigramm, US-Leber).

### Nach den neuen Erkenntnissen Indikation zu sekundären therapeutischen Maßnahmen:

Vom 2/88 bis 07.04.1988 nur Timunox-Monotherapie: 2 - 3 Ampullen Timunox 100 s.c. je nach Blutbildstatus und Status der funktionellen Subgruppen der Lymphozyten (Gesamt-T-Zellen, Helfer-, Suppressorzellen und NK-Zellen.

07.04.1988: Deutliche Besserung der allgemeinen Immunantwortfähigkeit auf Recall-Antigene (V). Deutlich meßbarer Effekt der Thymus/Timunox-Monotherapie auf die tumorassoziierte Immunreaktion im LMI-Test, jedoch ohne das Enhancement/Immuntoleranz durchbrechen zu können.

20.04. bis 29.06.1988: 1. Mitogene Immunstimulation. Auf dieser Weise, Kombination des Prüfpräparates (150348), eines Mistelextraktes mit standardisierten Mistellektinen I + II + III und mit standardisierter mitogener Aktivität/biologischer Aktivität, hochgradige Verstärkung der allgemeinen Immunantwort und Durchbruch des Enhancement, Immuntoleranz sowohl gegen Primärtumor, wie gegen Lymphknotenmetastasen-Gewebe als Antigenquelle (Gefrierschnittechnik) für den LMI.

DER TUMORMARKERVERLAUF (Ca-15-3, MCA, TPA) vom 21.12.1987, zum 2/88, zum 07.04./20.04.1988 und zum 29.06.1988 belegen, daß während der mitogenen Immunstimulation, durch diese alleinige Therapie, innerhalb von 6 - 8 Wochen zu einem deutlichen, signifikanten Abbau vom Tumormaterial kommt, hier um 30%).

VERMERK: Beim metastasierenden Mammakarzinom, bei begonnener Progression wird jedoch bevorzugt die Hormon-Immuntherapie und vor allem der Chemo-Immuntherapie, weil dies hocheffektiver ist, als die alleinige Chemotherapie, bzw. die alleinige Immuntherapie (mitogene Immunstimulation). Hier kann z. Z. noch keine allg. Empfehlung gegeben werden, die therapeut. Entscheidungen müssen noch individuell fallen!

### Patientin ME1015 Anlage 3 75-jährige Mammakarzinompatientin

Mammakarzinom, Primärtherapie im 2/85. Damals PT3, N4, Mo, Primärtumordurchmesser über 5,0 cm, 22 befallene Lymphknoten. Estrogen- und Progesteron-Rezeptoren positiv. Cytosol-CEA-Konzentration 8,7 ng/mg Tumorprotein. Serum-CEA präop. 47,0 ng/ml. Postop. 28,0 ng/ml, nach 2 1/2 Cyclen CMF 22,0 ng/ml, nach Wirksamwerden der adj. Tamoxifen-Therapie im Herbst 1985 28,0 ng CEA/ml.

Primärtherapie: Ablatio mammae, 2 1/2-Cyclen CMF, danach durchgehende Tamoxifen-Therapie ab 6/85 bis zum Unwirksamwerden. Im Zeitraum 11/85 bis 3/88, d. h. über 2 1/2 Jahre stabiler Serum-CEA-Werte zwischen 27 bis 32,0 ng CEA/ml. Vorübergehender treppenförmiger CEA-Anstieg nur während/nach von wiederholten schweren, fieberhaften Cystopyelitiden, ohne jedoch Immunparameter während dieser Krankheiten objektivieren zu können.

Ab 3/88 bis 7/88 beginnender, exponentieller Serum-CEA-Anstieg/-Progression bis 48 ngCEA/ml. Aus diesem Grunde Bestellung zur Therapieumstellung/Erweiterung:

Am 01.08.1988 Serum-CEA bereits 68 ng/ml (EIA-Hoffmann-La-Roche). Therapie und weiterer Verlauf siehe nachstehend.

### Hier Demonstration der Veränderung der tumorassoziierten Immunität:

26.02.1985 LMI-Test am 14. postop. Tag.

20.05.1985 LMI-Test nach 2 1/2 Cyclen CMF. Interpretation:

Nach Entfernung der Haupttumormasse und möglicherweise nach Eliminierung von zirkulierenden Immunkomplexen, die die Grundlage von Suppressor-Aktivitäten, wie das Enhancement bilden können, postive tumorassoziierte Immunreaktion gegen Primärtumor- und Lymphknoten-metastasengewebe mit Inhibition bei guter Allgemeinimmunität (V).

Vermerk: Hier wäre, würde ggf. eine Indikation für ASI (ASI = aktiv spezifische Immunstimulation, daß heißt eine geeignete Vakzination) sein. Durch die zusätzliche postive Verstärkung der gegebenen tumorassoziierten Immunität, ohne schlechte Prognose, wie Enhancement, könnte der weitere Verlauf der Krankheit positiv, günstig beeinflußt werden, insbesondere in Kombination mit Tamoxifen.

01.08.1988: Hochgradiges Enhancement im Rahmen der begonnenen Tumorprogression. Verschlechterung der allgemeinen Immunität. Cyanose der Lippen, möglicherweise infolge beginnender Lungenkarzinose und auf Effekt von zirkulierenden Immunkomplexen. Im weiteren Verlauf (Die Patientin erhielt ab 01.08.1988 insgesamt 3 Cyclen mitogene Immunstimulation, in der Standarddosierung: 1 x 1 Ampulle Plenosol^{R}-Stärke II i. v. pro Woche und 2 bis 1 Ampulle Timunox 100 s. c. Der Serum-CEA-Spiegel sank jeweils während der 6-wöchigen Immuntherapiecyclen um 30 bis 40%, von 41 ng/ml auf 28 ng/ml und dann auf 16 ng/ml bis 4/89. Serum-CEA-Spiegel normal bis 5 ng/ml. In den Therapiepausen, zwischen den Immuntherapiecyclen wurde Hormontherapie (Tamoxifen, bzw. hochdosiertes Medroxyprogesteronazetat), bzw. Chemotherapie (Mitomycin-C) ohne Erfolg, ohne therapeutischen Effekt klinisch eingesetzt. An diesem Behandlungsfall kann die therapeutische Potenz der mitogenen Immunstimulation dramatisch belegt werden: die Immuntherapie ist hocheffektiv, obwohl alle sonstigen schulmedizinischen therapeutischen Möglichkeiten bereits unwirksam geworden sind) deutlich Rosigwerden der Lippen. Klinisch-röntgenologisch zu diesem Zeitpunkt noch no-Evidence-of-diseasis. Trotzdem Therapieumstellung/Ergänzung, da die durchgeführte Multiparameter-Studie zeigt, daß diese Befundkonstellation ein Todesurteil bedeutet, darstellt und passives Verhalten/Abwarten heute ethisch nicht mehr zu verantworten ist.

19.09.1988 Durch mitogene Immunstimulation (im Rahmen der Therapieumstellung) Durchbruch des Enhancement/Immuntoleranz und mächtige Verstärkung der allgemeinen Immunität (V-Zeichen). CEA am 19.09.1988 ca. 41 ng/ml.

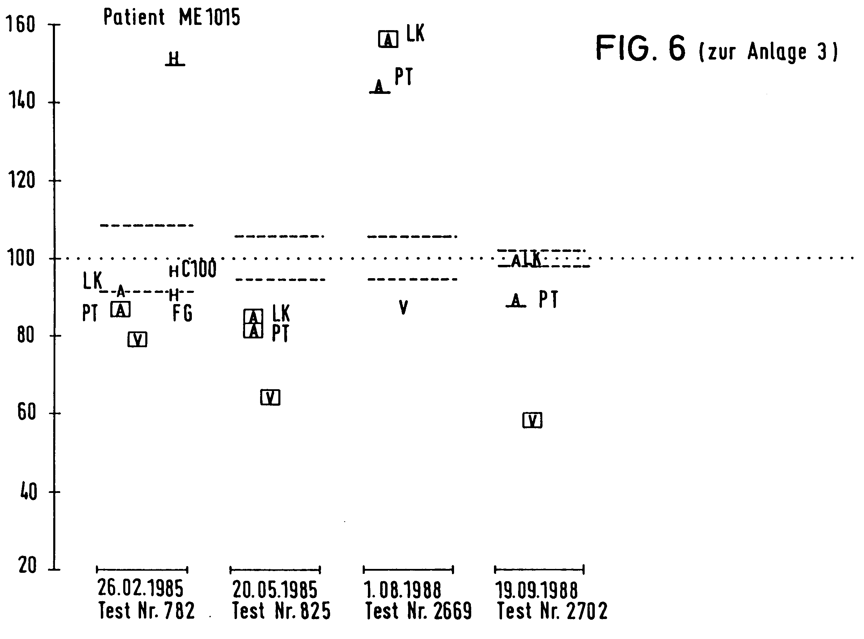

### Patientin ME1015 Anlage 4 75-jährige Mammakarzinompatientin (Weiterer Krankheits- und Behandlungsverlauf zum Fall, der in der Anlage 3 und in Abbildung 6 beschrieben worden ist)

Ursprünglich, vor dem Beginn der Immuntherapie/mitogene Immunstimulation am 01.08.1988 betrug der CEA-Serumspiegel (=Tumormarker) 68,0 ng/ml. Dies ist ein hochpathologischer Wert: normal ist bis 5,0 ng CEA/ml.

Im Zeitraum 1.8.88 bis November 1989 absolvierte die Patientin insgesamt 5 x 6 Wochen mitogene Immunstimulation mit wöchentlich 1 x 20 Klinischen Einheiten mitogener Induktor aus Mistelextrakt i.v. Bolus und wöchentlich 2 x 50 mg TP 5 (= Timunox^{R}) s.c. Bis zum November 1989 sank der Serum-CEA-Spiegel auf 6,5 ng/ml. Während einer weiteren 6-Wochenkur konnte der CEA-Serumspiegel nicht weiter gesenkt werden. Es erfolgte eine Therapiepause. Während dieser Therapiepause kam es bereits nach einer kurzen Zeit zu stetigem und kontinuierlichem Wiederanstieg der Tumormarker-Serumspiegel CEA und Ca-15-3. (Ca-15-3-Verlauf hier nicht dargestellt).

Am 21.03.90 erreichte der Serum-CEA-Spiegel erneut den Wert von 14,8 ng/ml.

Am 27.03.90 wurde im LMI-Test die tumorassoziierte Immunität erneut überprüft (siehe Abbildung 7). V = Darstellung der Immunreaktion gegen die Recallantigene Streptokinase/Streptokinase zur Erfassung der allgemeinen Immunitätslage. A = Darstellung der tumorassoziierten Immunreaktion gegen autologes Tumorgewebe: PT = Primärtumor/LK = Lymphknotenmetastase. Hier wurde noch die alte 20-mü-Gefrierschnitt-Technik des LMI-Testes als Antigenquelle angewandt.

Vom 27.03.90 bekam die Patientin erneut eine mitogene Immunstimulationskur, wie oben beschrieben. Entsprechend der sehr schwachen, nur einmal signifikanten tumorassoziierten Reaktion gegen LK-Tumorgewebe, bewirkte die Immuntherapie nur einen Progressionsstillstand, wie es aus den wöchentlich bestimmten CEA-Serumspiegeln zu entnehmen ist.

| | | |
|---|---|---|
| Serum-CEA-Spiegel | am 27.03.90 | 14,80 ng/ml |
| | am 16.04.90 | 14,07 ng/ml |
| | am 23.04.90 | 14,46 ng/ml |
| | am 30.04.90 | 17,06 ng/ml |
| | am 07.05.90 | 14,32 ng/ml |
| | am 12.05.90 | 14,75 ng/ml |

Hieraus läßt sich die Erkenntnis ableiten, wie aus mehreren, ähnlich gelagerten Behandlungsfällen,
erstens, daß die Wirksamkeit der mitogenen Immunstimulation von der Qualität und Stärke der ex-vivo-in-vitro meßbaren tumorassoziierten Immunität abhängig ist und
zweitens, daß in Zukunft diese Form der Krebsimmuntherapie vorzugsweise mit einer anderen Form der Tumorimmuntherapie, mit einer Autovakzinationsmethode, wie der aktiv-spezifischen Immuntherapie (= ASI), kombiniert werden muß, um Dauervollremissionen bzw. Vollheilungen erzielen zu können.

### Patientin ME1015 Anlage 5 75-jährige Mammakarzinompatientin (Weiterer Krankheits- und Behandlungsverlauf zum Fall, der in den Anlagen 3+4 und in den Abbildungen 6+7 beschrieben worden ist)

- Hier:: Demonstration des zusätzlichen therapeutischen Effektes und der Wirkungsweise der mitogenen Immunstimulation, wenn man die 2-er Medikamentenkombination (mitogener Induktor aus Viscum-album-Extrakt + synthetisches Thymuspeptid/TP5 (= Timunox^{R}) mit low-dose-Interleukin-2 in eine 3-er Medikamentenkombination erweitert.

Ab 14.05.90 (Montag) erhielt die Pat. folgende Therapie:
- wöchtentlich 1 x 20 Klinische Einheiten mitogener Induktor 150348 aus Viscum-album-Extrakt i.v. Bolus.(x)
- wöchentlich 3 x 50 mg TP 5 (= Timunox^{R}) s.c. Montag, Mittwoch, Freitag
- wöchtentlich 3 x 3,0 Millionen Interleukin-2/gm Körperoberfläche, d.h. hier jeweils 5,0 Millionen IE Proleukin^{R} (Hersteller und Vertrieb: Eurocetus-GmbH, D-6000 Frankfurt a.M.) als Dauerinfusion über 4 Stunden jeweils am Montag, Mittwoch, Freitag einer Woche
insgesamt über 2 Wochen.

| | | | |
|---|---|---|---|
| Serum-CEA-Spiegelverlauf | am 12.05.90 | 14,40 ng/ml | (in Wiederholung) |
| | am 14.05.90 | 14,75 ng/ml | |
| | am 21.05.90 | 10,10 ng/ml | |
| | am 28.05.90 | 7,05 ng/ml | |

Zusammensetzung und Veränderungen der funktionellen Subgruppen der Lymphozyten:

| | am 14.05. | am 21.05. | am 28.05. | Normalwerte |
|---|---|---|---|---|
| Gesamtlymphozyten | 1150/cmm | 1350/cmm | 1470/cmm | über 1200/cmm |
| Gesamt-T-Zellen | 68 % | 73 % | 77 % | 58 - 73 % |
| B-Zellen | 25 % | 21 % | 15 % | 11 - 17 % |
| Helfer-Zellen | 32 % | 36 % | 42 % | 20 - 45 % |
| Supressorzellen | 25 % | 20 % | 18 % | 12 - 25 % |
| H/S-Zellen-Ratio | 1,28 % | 2,0 % | 2,33 % | 1,40 - 2,00 u. höher |
| NK-Zellen | 9 % | 10 % | 14 % | 7 - 15 % |
| Aktivierte T-Zellen | 14 % | 21 % | 24 % | 2 - 12 % |
| CTL= cytotoxische | | | | |
| T-Zellen | 12 % | 26 % | 31 % | 2 - 10 % |
| Interleukin-2-Rezeptor-positive | | | | |
| Natural-Killer-Zellen | 1 % | 2 % | 5 % | 1 - 4 % |
| Interleukin-2-Rezeptor-positive | | | | |
| T-Zellen insgesamt | 17 % | 27 % | 38 % | 2 - 10 % |

Vermerk: - Beim mitogenen Induktor 150348 handelt es sich um einen immunpharmakologisch standardisierten Mistelextrakt, bezogen auf Lektingehalt und auf die Fähigkeit in-vivo im Tierversuch die T-zellenabhängige, adoptive, zelluläre und antikörperabhängige zelluläre Immunreaktion gegen Recallantigene, wie Tuberkulin, Streptokinase/Streptodornase im Leukozytenmigrationsinhibitionstest (LMI) signifikant verstärken zu können.
- Der mitogene Induktor 150348 ist ein Handelspräparat des Serobac^{R}-Institutes für Onkologie, D-5650 Solingen 11, Postfach 11 1053. Im Präparat ist das Verhältnis der Mistellektine zu den sonstigen Wirk- und Ballaststoffen um den Faktor 10 - 25 besser als in den üblichen Handelspräparaten deutschsprachiger Länder. Weiterhin ist das Präparat praktisch ohne jegliche Nebenwirkungen i.v. systematisch applizierbar.

Die hier an diesem Fallbeispiel erhobene und dargelegte Wirkungsweise der zusätzlich low-dose-applizierten Interleukin-2 in der Medikamentenkombination zur mitogenen Immunstimulation ist ein therapietypischer Effekt. Die zusätzliche Gabe von low-dose-Interleukin-2 verstärkt additiv die bereits hochwirksame 2-er-Medikamentenkombination.

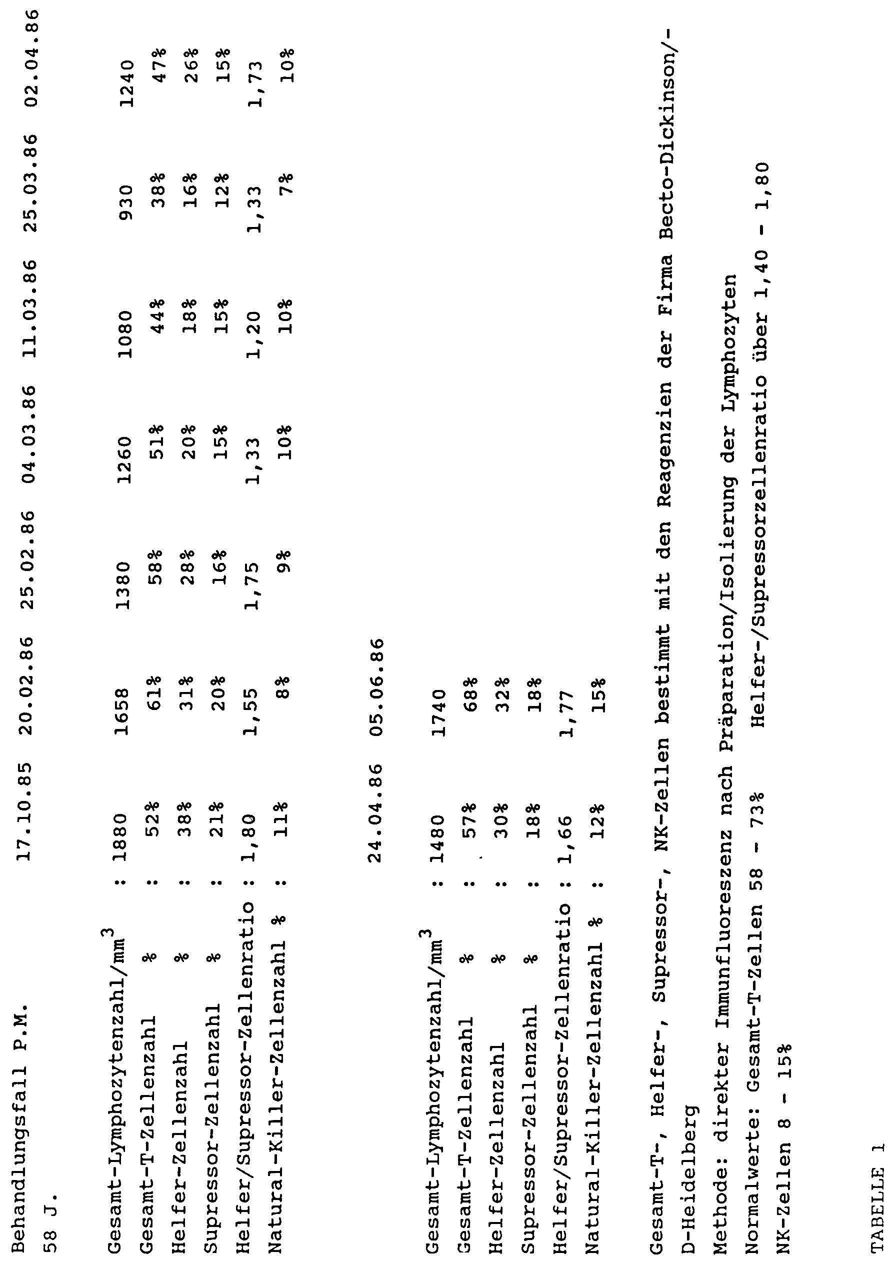

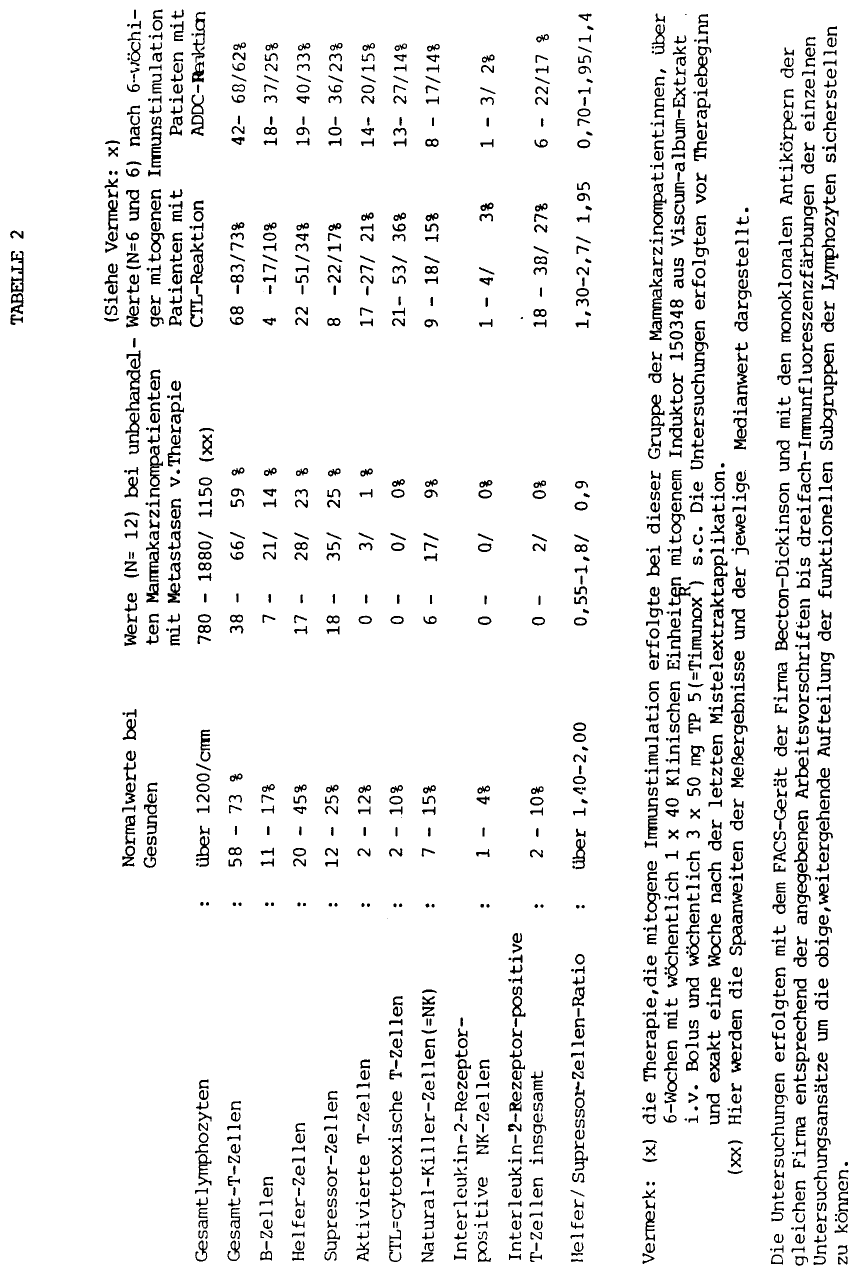

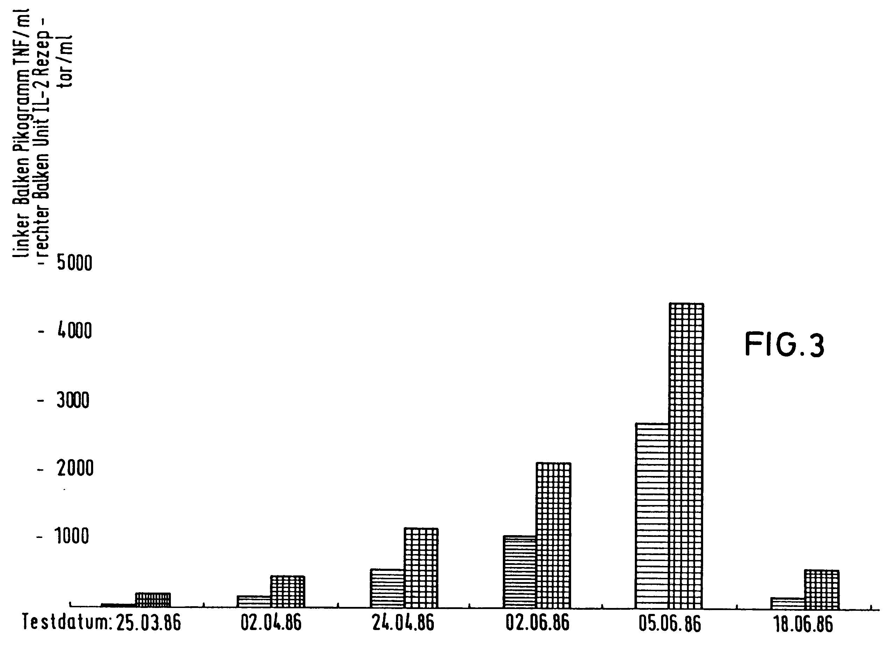

### Erläuterungen zur Figur 3:

### PATIENT-NR: 0828 P.M. weiblich, 58 J. Diagnose: metastasierendes Mammakarzinom

Demonstration des Zusammenhanges zwischen der Reaktionsstärke der T-Zellabhängigen, antikörpergesteuerten, komplementabhängigen, tumorassoziierten Immunität, gemessen im Leukozytenmigrationsinhibitionstest und dem mitogen in-vivo induzierten Lymphokinserumspiegel, hier: Tumornekrosefaktor und Interleukin-2-Rezeptorserumspiegel im Serum der Brustkrebspatientin, gemessen mit Hilfe des BIOKINB™ TNF-Test Kit und des CELLFREE™Interleukin-2-Receptor Test Kit der Firma T-Cell Sciences, Inc., 840 Memorial Drive Cambridge, MA.USA.
Zur Messung der T-Zellabhängigen adoptiven tumorassoziierten Immunität im Leukozytenmigrationsinhibitionstest (LMI-Test) wurde als autologe Tumorantigenquelle prozessierte Cytosolreste (lösbare Tumorantigenfraktion) und Tumorsedimentreste (nicht lösbare Zellmembran/Zellwand-Antigenfraktion) eingesetzt.
MITOGENE IMMUNSTIMULATION ist eine Innovation des SEROBAC^{R}-Institutes für Onkologie, D-5650 Solingen 11, Pf. 111053.

Die hochgradige in-vivo-Induktion von TNF und Interleukin-2R wurde an der Toxizitätsgrenze erreicht mit der Prüfsubstanz: 150348 von Serobac^{R}. Hier handelt es sich um ein pflanzliches Lektingemisch, das in-vivo weniger toxisch ist als PHA oder Con-A aus der Gruppe der bekannten in-vitro-Lymphokininduktoren.

Höchster Serumspiegel von TNF bei Blutspender: 67 pg/ml (N=136)
Höchster Serumspiegel von IL-2R bei Blutspender: 477 U/ml (N=174)

## Patentansprüche

1. Kombinierte Verwendung von mitogen immunstimulierenden und thymomimetischen Substanzen zur Herstellung von Medikamenten zur Tumortherapie mit kontrolliertem und reguliertem Immunsystem.

2. Kombinierte Verwendung gemäß Anspruch 1, dadurch gekennzeichnet, daß als mitogen immunstimulierende Substanzen Lektine oder lektinhaltige Extrakte eingesetzt werden.

3. Kombinierte Verwendung gemäß Anspruch 1 oder 2, dadurch gekennzeichnet, daß als mitogen immunstimulierende Substanz lektinhaltige Mistelextrakte eingesetzt werden.

4. Kombinierte Verwendung gemäß einem der Ansprüche 1 bis 3, dadurch gekennzeichnet, daß als thymomimetische Substanzen Thymusextrakte, synthetische Thymuspeptide oder thymomimetisch aktive Peptidfragmente eingesetzt werden.

5. Kombinierte Verwendung gemäß einem der Ansprüche 1 bis 4, dadurch gekennzeichnet, daß die mitogen immunstimulierenden Substanzen in solch einer Konzentration im Medikament vorliegen, daß damit eine meßbare Freisetzung von Lymphokinen erfolgt.

6. Kombinierte Verwendung gemäß einem der Ansprüche 1 bis 5, dadurch gekennzeichnet, daß zusätzlich Interleukin-2 in niedriger Konzentration eingesetzt wird.

7. Kombinationspräparate zur Tumortherapie mit kontrolliertem und reguliertem Immunsystem enthaltend
a) mitogen immunstimulierende Substanzen und
b) thymomimetische Substanzen.

8. Kombinationspräparate gemäß Anspruch 7, dadurch gekennzeichnet, daß sie als mitogen immunstimulierende Substanzen Lektine oder lektinhaltige Extrakte enthalten.

9. Kombinationspräparate gemäß Anspruch 7 oder 8, dadurch gekennzeichnet, daß sie als thymomimetische Substanzen Thymusextrakte, synthetische Thymuspeptide oder thymomimetisch aktive Peptidfragmente enthalten.

10. Kombinationspräparate gemäß Ansprüchen 7 bis 9, dadurch gekennzeichnet, daß sie zusätzlich Interleukin-2 in niedriger Konzentration enthalten.

11. Verfahren zur verbesserten Kontrolle des Immunsystems vor und während der Tumortherapie mit einer mitogen immunstimulierenden Substanz und einer thymomimetischen Substanz, dadurch gekennzeichnet, daß beim Leukozytenmigrationsinhibitionstest (LMI) und/oder Leukozytenadhärenzinhibitionstest (LAI) mit mehreren verschieden großen Antigendosen getestet wird, um die T-zellabhängige, adoptive, tumorassoziierte Immunität ex-vivo-in-vitro zu messen.

## Claims

1. The combined use of mitogenically immunostimulating and thymomimetic substances for the preparation of medicaments for tumor therapy involving a controlled and regulated immune system.

2. The combined use according to claim 1, characterized in that lectins or lectin-containing extracts are employed as said mitogenically immunostimulating substances.

3. The combined use according to claim 1 or 2, characterized in that lectin-containing mistletoe extracts are employed as said mitogenically immunostimulating substances.

4. The combined use according any of claims 1 to 3, characterized in that thymus extracts, synthetic thymus peptides or thymomimetically active peptide fragments are employed as said thymomimetic substances.

5. The combined use according any of claims 1 to 4, characterized in that said mitogenically immunostimulating substances are present in the medicament in such a concentration as will result in a measurable release of lymphokines.

6. The combined use according any of claims 1 to 5, characterized in that interleukin-2 is additionally employed in a low concentration.

7. Combined preparations for tumor therapy involving a controlled and regulated immune system, containing
a) mitogenically immunostimulating substances, and
b) thymomimetic substances.

8. Combined preparations according to claim 7, characterized by containing lectins or lectin-containing extracts as said mitogenically immunostimulating substances.

9. Combined preparations according to claim 7 or 8, characterized by containing thymus extracts, synthetic thymus peptides or thymomimetically active peptide fragments as said thymomimetic substances.

10. Combined preparations according to claims 7 to 9, characterized by additionally containing interleukin-2 in a low concentration.

11. A method for improved control of the immune system prior to and in the course of tumor therapy using a mitogenically immunostimulating substance and a thymomimetic substance, characterized in that in leucocyte migration inhibition test (LMI) and/or leucocyte adherence inhibition test (LAI), testing is performed with a number of different antigen dosage quantities in order to measure ex vivo/in vitro the T-cell dependent adoptive tumor-associated immunity.

## Revendications

1. Utilisation combiné des substances immunostimulantes mitogènes et des substances thymomimétiques pour la préparation de médicaments pour la thérapie des tumeurs dans laquelle le système immun est contrôlé et réglé.

2. Utilisation combiné selon la revendication 1, caractérisée en ce que des lectines ou des extraits contenant des lectines sont utilisés comme lesdites substances immunostimulantes mitogènes.

3. Utilisation combiné selon l'une des revendications 1 et 2, caractérisée en ce que des extraits de gui contenant des lectines sont utilisés comme lesdites substances immunostimulantes mitogènes.

4. Utilisation combiné selon l'une des revendications 1 à 3, caractérisée en ce que des extraits de thymus, des peptides de thymus synthétiques ou des fragments de peptide thymomimétiquement actives sont utilisés comme lesdites substances thymomimétiques.

5. Utilisation combiné selon l'une des revendications 1 à 4, caractérisée en ce que lesdites substances immunostimulantes mitogènes sont présentes dans le médicament dans une concentration qui mène à une libération mesurable de lymphokines.

6. Utilisation combiné selon l'une des revendications 1 à 5, caractérisée en ce que l'interleukine 2 est utilisée en plus dans une faible concentration.

7. Préparations combinées pour la thérapie des tumeurs dans laquelle le système immun est contrôlé et réglé, contenant
a) des substances immunostimulantes mitogènes, et
b) des substances thymomimétiques.

8. Préparations combinées selon la revendication 7, caractérisée en ce qu'elles contiennent des lectines ou des extraits contenant des lectines comme lesdites substances immunostimulantes mitogènes.

9. Préparations combinées selon l'une des revendications 7 et 8, caractérisée en ce qu'elles contiennent des extraits de thymus, des peptides de thymus synthétiques ou des fragments de peptide thymomimétiquement actives comme lesdites substances thymomimétiques.

10. Préparations combinées selon l'une des revendications 7 à 9, caractérisée en ce qu'elles contiennent en plus l'interleukine 2 dans une faible concentration.

11. Procédé pour le contrôle amélioré du système immun avant et pendant la thérapie des tumeurs avec une substance immunostimulante mitogène et une substance thymomimétique, caractérisée en ce que le test d'inhibition de la migration des leucocytes (LMI) et/ou le test d'inhibition de l'adhérence des leucocytes (LAI) sont effectués avec plusieurs doses d'antigène à quantité différente pour mesurer de façon ex vivo/in vitro l'immunité associée aux tumeurs adoptive dépendante des lymphocytes T.
